# EUROPÄISCHE PATENTSCHRIFT

(11) **EP 2 923 670 B1**
(45) Veröffentlichungstag und Bekanntmachung des Hinweises auf die Patenterteilung: **10.05.2017**
(21) Anmeldenummer: 14161631.8
(22) Anmeldetag: 26.03.2014
(51) Int. Cl.: A61C 9/00, A61C 13/00, G01B 11/00

(54) **Dreidimensionaler Körper**
Three-dimensional body
Corps tridimensionnel

(43) Veröffentlichungstag der Anmeldung: 30.09.2015
(73) Patentinhaber: Krischollek, Michael, 12589 Berlin (DE); Tridenta Dentaltechnik GmbH, 10407 Berlin (DE)
(72) Erfinder: Michael, Krischollek, 12589 Berlin (DE); Ilmer, Viola, 10407 Berlin (DE); Jasper, Frank, 10407 Berlin (DE)
(74) Vertreter: Schneider, Henry

(56) Entgegenhaltungen:
- EP-A1- 2 719 357
- DE-B3-102012 201 193
- US-A1- 2001 038 705

## Beschreibung

### Gebiet der Erfindung

Die Erfindung betrifft ein Verfahren um zwei oder mehrere Darstellungen eines Kiefers mit Ober- und Unterkiefer zueinander in Bezug zu setzen.

### Hintergrund der Erfindung

Auf dem Gebiet der Kieferorthopädie, Zahntechnik und Zahnimplantation kommen zunehmend computergestützte Therapiepläne zum Einsatz. Diese werden verwendet um Größe, Abmessungen und Integration von Zahnersatz, beispielsweise Inlays, Brücken oder Kronen, zu berechnen. Insbesondere bei der Herstellung von Implantaten für einzelne oder mehrere Zähne bis hin zu vollständigen Gebissen wird computergestütztes Design (englisch: Computer-Aided-Design, CAD) verwendet. Gestalt und Positionierung des Implantats werden in der Regel basierend auf Aufnahmen des Kiefers oder dreidimensionalen Scans von Gipsmodellen, die nach Kieferabdrücken hergestellt wurden, berechnet. Die Implantate oder Brücken werden am Computer entworfen und mittels computergestützten Frästechniken hergestellt. Je nach medizinischer Anwendung kommen dabei verschiedene dentaldiagnostische Aufnahmetechniken zum Einsatz, wie beispielsweise Panoramaröntgen, Aufnahmen von Zahnfilme einzelner Zähne oder Zahnreihen, Intraoralscan, Computertomographie und drei-dimensionale Volumentomographie. Durch die unterschiedlichen Aufnahmetechniken, Maßstäbe, Aufnahmewinkel und verschiedenen abgebildeten Teilbereichen des Kiefers bzw. Gebisses ist es jedoch kaum möglich die erhaltenen Aufnahmen untereinander und insbesondere mit früher aufgenommenen Darstellungen zuverlässig zu vergleichen. Dies wäre jedoch wünschenswert, um die Veränderungen des Kiefers und des Zahnstatus ermitteln zu können. Über die Zeit verändern sich sowohl Kiefer als auch Zahnstatus, gegebenenfalls bis hin zum Verlaust einzelner oder mehrerer Zähne. Bei der Behandlung von Zahndefekten wird derzeit überwiegend darauf abgezielt durch zahnerhaltende Maßnahmen oder Zahnersatz das Gebiss gemäß dem Ist-Zustand zu erhalten bzw. zu vervollständigen. Dabei werden Veränderungen des Kiefers jenseits des unmittelbar zu behandelten Defekts in der Regel außer Acht gelassen, da sich retrospektiv kaum nachvollziehen lässt wie sich der Ist-Zustand vom ursprünglichen Zustand des Kiefers unterscheidet. Mit dem Kiefer verändert sich jedoch regelmäßig auch die Okklusion und Artikulation des Ober- und Unterkiefers, wodurch craniomandibuläre Dysfunktionen entstehen. Bei zahntechnischen Maßnahmen, die sich ausschließlich am Ist-Zustand orientieren können derartige Veränderungen nicht ausgeglichen werden, so dass trotz Behandlung Beeinträchtigungen des Patienten bleiben können.

Es besteht daher ein Bedarf nach einem Verfahren um unterschiedliche dentaldiagnostische Aufnahmen zu vergleichen und auszuwerten, so dass ein ursprünglicher, möglichst idealer Zahnstatus wieder hergestellt werden kann.

### Zusammenfassung der Erfindung

Die vorliegende Erfindung betrifft ein Verfahren umfassend die Schritte
a) Bereitstellen einer ersten medizintechnischen Darstellung eines Kiefers mit Oberkiefer und Unterkiefer,
   a₁ Erfassen eines anatomisch stabilen rechten Direktionspunkts A, eines anatomisch stabilen linken Direktionspunkts B und eines anatomisch stabilen palatinal im Frontzahnbereich gelegenen Induktionspunkts C des Oberkiefers in der ersten Darstellung,
   a₂ Erfassen eines anatomisch stabilen rechten Direktionspunkts A', eines anatomisch stabilen linken Direktionspunkts B' und eines anatomisch stabilen labial gelegenen Induktionspunkts C' des Unterkiefers in der ersten Darstellung,
   a₃ Bilden eines ersten geometrischen Körpers mit einer ersten Fläche zwischen den Punkten A, B, und C' und einer zweiten Fläche zwischen den Punkten A', B' und C in der ersten Darstellung,
b) Bereitstellen einer zweiten medizintechnischen Darstellung des Kiefers,
   b₁ Erfassen eines anatomisch stabilen rechten Direktionspunkts A, eines anatomisch stabilen linken Direktionspunkts B und eines anatomisch stabilen palatinal im Frontzahnbereich gelegenen Induktionspunkts C des Oberkiefers in der zweiten Darstellung,
   b₂ Erfassen eines anatomisch stabilen rechten Direktionspunkts A', eines anatomisch stabilen linken Direktionspunkts B' und eines anatomisch stabilen labial gelegenen Induktionspunkts C' des Unterkiefers in der zweiten Darstellung,
   b₃ Bilden eines zweiten geometrischen Körpers mit einer ersten Fläche zwischen den Punkten A, B, und C' und einer zweiten Fläche zwischen den Punkten A', B'und C in der zweiten Darstellung, und
c) In Bezug setzen der ersten und zweiten Darstellung indem der erste geometrische Körper und der zweite geometrische Körper als gemeinsame Referenz dienen, wobei die Schritte a - c mit einem Computerprogramm durchgeführt werden.

Die Erfindung betrifft ferner ein Computerprogramm aufweisend einen Programmcode um einen Computer zu steuern, um die Schritte des erfindungsgemäßen Verfahrens auszuführen, wenn das Computerprogramm auf einem Computer ausgeführt wird.

Des Weiteren betrifft die Erfindung eine medizintechnische Darstellungsvorrichtung für die Wiedergabe von mindestens zwei medizintechnischen Darstellungen eines Kiefers, mit einer Datenspeicherungsvorrichtung, die eine erste und eine zweite medizintechnische Darstellung des Kiefers speichert, einer Datenverarbeitungsvorrichtung, die einen ersten geometrischen Körper in der ersten Darstellung und eine zweiten geometrischen Körper in der zweiten Darstellung bildet, und einer Graphikverarbeitungsvorrichtung, die die erste und zweite Darstellung wiedergibt, wobei die erste und zweite Darstellung übereinstimmend angeordnet werden indem der erste geometrische Körper und der zweite geometrische Körper als gemeinsame Referenz dienen, dadurch gekennzeichnet, dass der erste und zweite geometrische Körper jeweils gebildet werden mit einer ersten Fläche zwischen einem anatomisch stabilen rechten Direktionspunkts A, einem anatomisch stabilen linken Direktionspunkts B und einem anatomisch stabilen palatinal im Frontzahnbereich gelegenen Induktionspunkts C des Oberkiefers und einer zweiten Fläche zwischen einem anatomisch stabilen rechten Direktionspunkts A', einem anatomisch stabilen linken Direktionspunkts B' und einem anatomisch stabilen labial gelegenen Induktionspunkts C' des Unterkiefers

### Kurzbeschreibung der Figuren

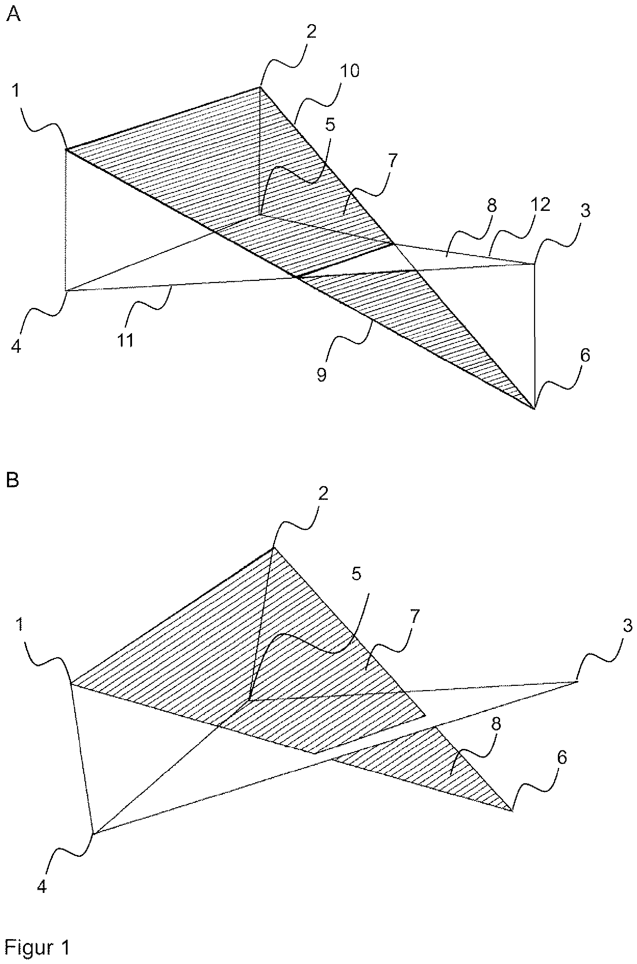

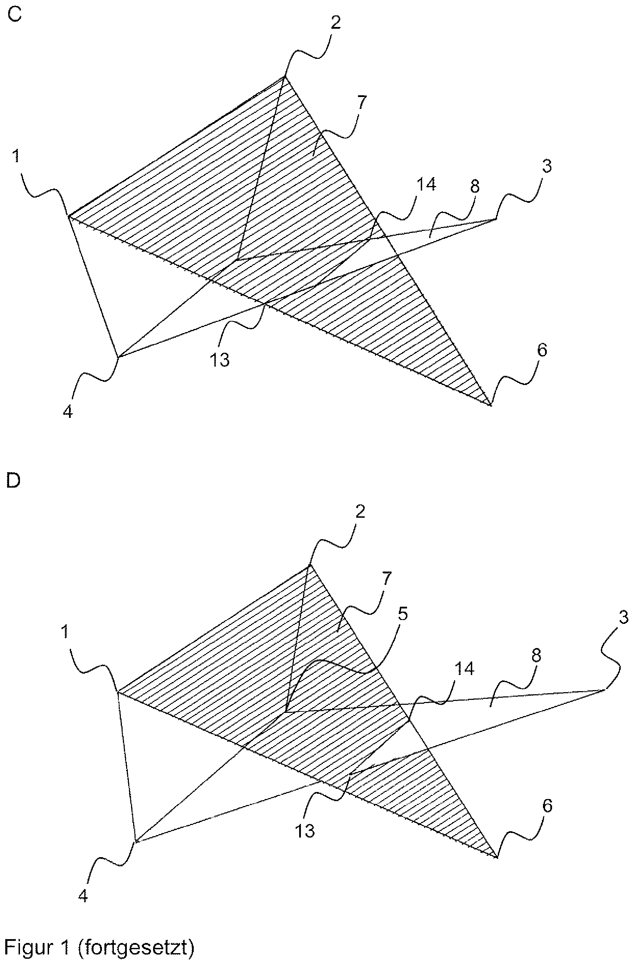

Figur 1 zeigt Darstellungen eines geometrischen Körpers, der gebildet wird durch zwei Flächen. Die erste Fläche (7) wird aufgespannt durch die Punkte A (1), B (2) und C' (6) und die zweite Fläche (8) durch die Punkte A' (4), B' (5) und C (3). Bei dem in Figur 1 A dargestellten geometrischen Körper schneiden sich die erste Fläche (7) und die zweite Fläche (8) an den Außenkanten [AC'] (9) und [A'C] (11) sowie in den Außenkanten [BC'] (10) und [B'C] (12). Bei dem in Figur 1B dargestellten geometrischen Körper tritt die erste Fläche (7) durch die zweite Fläche (8) hindurch, so dass die Längskanten der ersten Fläche (7) [AC'] (9) und [BC'] (10) die zweite Fläche (8) in den Durchtrittspunkten D₁ (13) und D₂ (14) schneiden. Bei dem in Figur 1C dargstellten geometrischen Körper tritt die zweite Fläche (8) durch die erste Fläche (7) hindurch, so dass die Längskanten der zweiten Fläche (8) [A'C] (11) und [B'C] (12) die erste Fläche (7) in den Durchtrittspunkten D₁ (13) und D₂ (14) schneiden. Bei dem in Figur 1D dargestellten geometrischen Körper schneiden sich die erste und zweite Fläche derart, dass die Längskante der ersten Fläche (7) [BC'] (10) die zweite Fläche (8) im Durchtrittspunkt D₂ (14) schneidet und die Längskante der zweiten Fläche (8) [A'C] (11) die erste Fläche (7) in dem Durchtrittspunkt D₁ (13) schneidet.

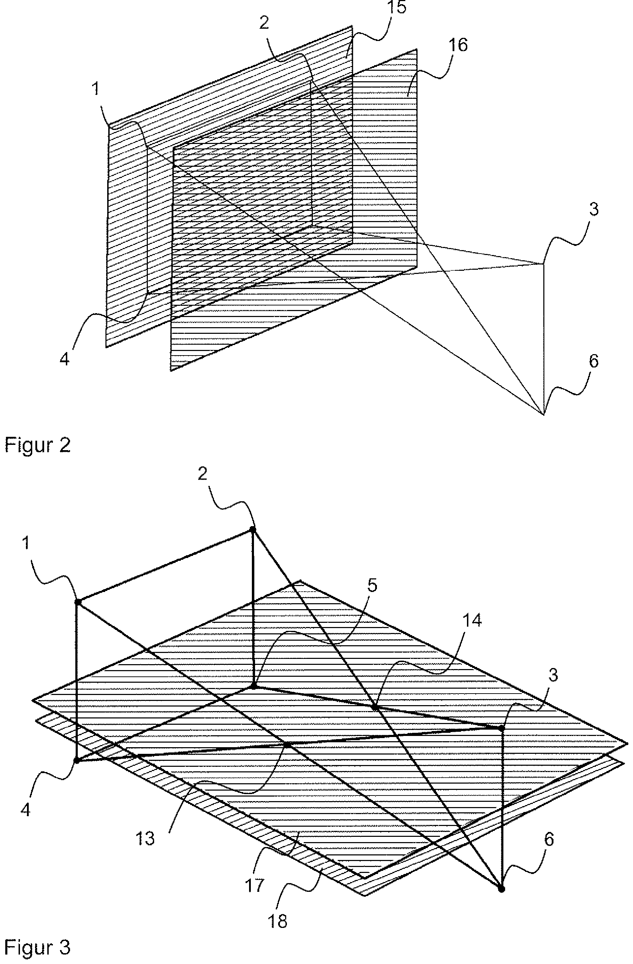

Figur 2 zeigt den in Figur 1A dargestellten geometrischen Körper mit einer ersten vertikalen Schnittebene Sᵥ⁰ (15) und einer weiteren parallel angeordneten Schnittebenen Sᵥ¹ (16).
Figur 3 zeigt den in Figur 1A dargestellten geometrischen Körper mit einer ersten horizontalen Schnittebene S_{H}⁰ (17) und einer weiteren horizontalen Schnittebenen S_{H}¹ (18), die parallel zu S_{H}⁰ angeordnet sind.

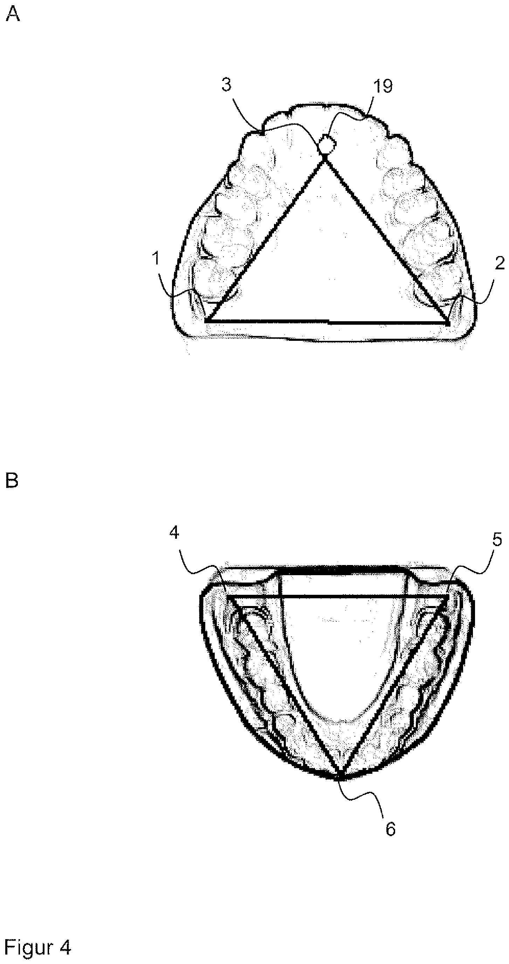

Figur 4 zeigt die cranialen Referenzpunkte nach Staub im Oberkiefer (A) und Unterkiefer (B). Der rechte Direktionspunkt A (1) liegt in der rechten Oberkieferhälfte an dem Punkt der Richtungsänderung der Linie, die sich von der Kieferkammmitte zum Hamulus pterygoideus fortsetzt, von konvex zu konkav. Der linke Direktionspunkt B (2) liegt an dem entsprechenden Punkt in der linken Oberkieferhälfte. Der Induktionspunkt C (3) ist der Schnittpunkt zwischen der Medianachse des Oberkiefers und der posterioren Umrisslinie der Papilla (19). Der rechte Direktionspunkt A' (4) liegt in der rechten Unterkieferhälfte an dem Punkt der Richtungsänderung von konvex zu konkav der mesio-distalen Verlaufskurve der Kammverbindungslinie am distalen Ende der Trigona retromolare. Der linke Direktionspunkt B' (5) liegt an dem entsprechenden Punkt in der linken Unterkieferhälfte. Der Induktionspunkt C' (6) des Unterkiefers ist der Schnittpunkt der Medianachse des Unterkiefers mit der Conclusionslinie, welche die Grenzlinie zwischen beweglicher und unbeweglicher Schleimhaut vor den Schneidezähnen bildet.

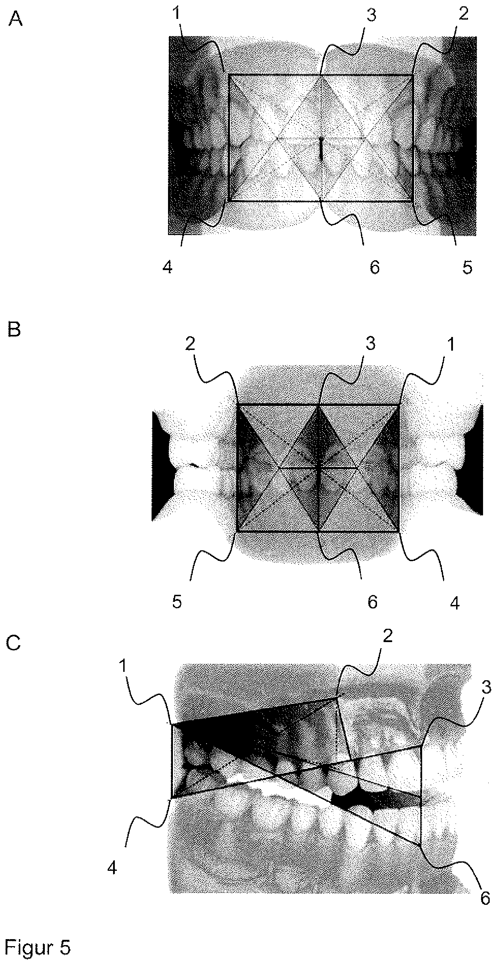

Figur 5 zeigt Aufnahmen eines Kiefers mit Zähnen, in dem ein geometrischer Körper angeordnet ist. Der Kiefer ist dargestellt von vorne (A), von hinten (B) und aus einer seitlichen Perspektive (C).

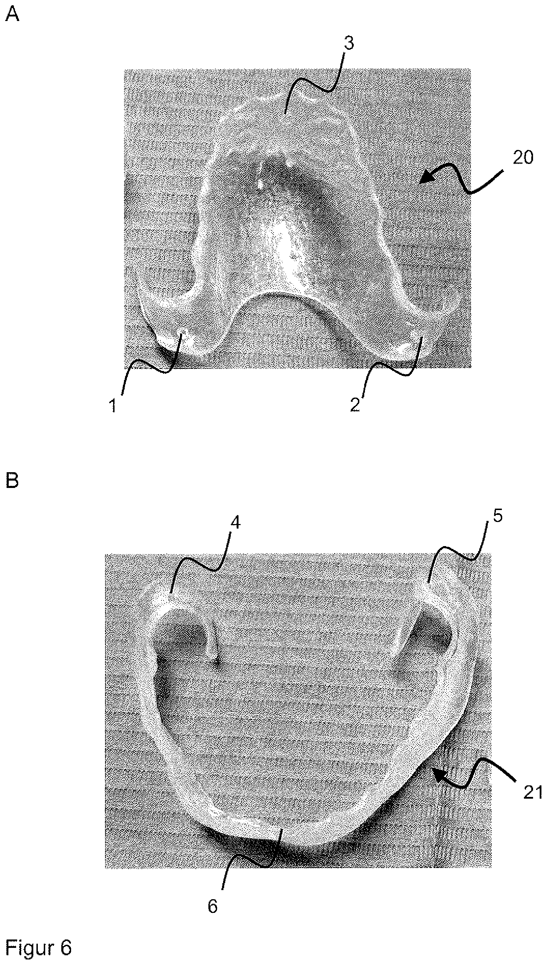

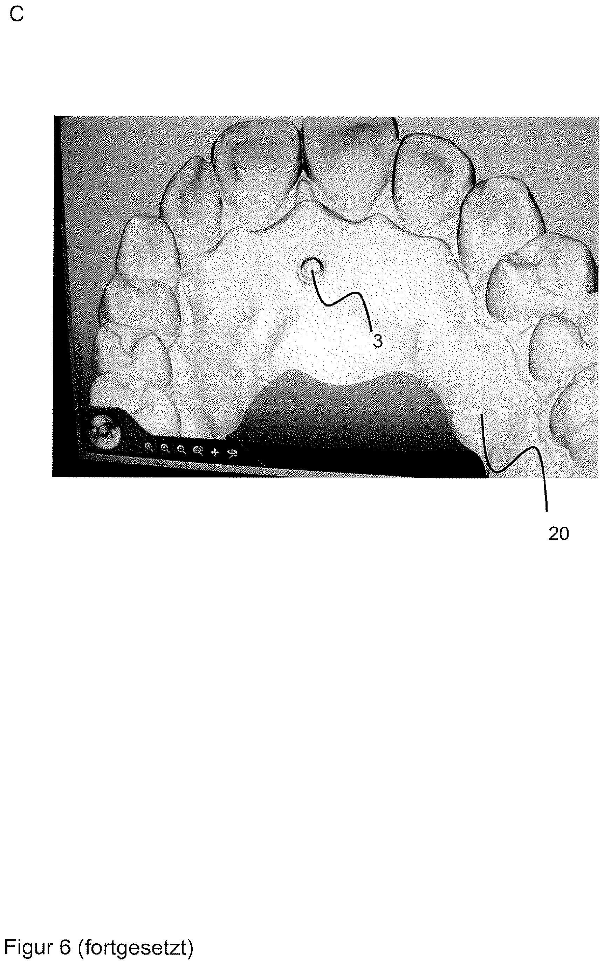

Figur 6A zeigt eine zahnreihenunabhängige Schiene für den Oberkiefer (A) und für den Unterkiefer (B). Auf der Oberkieferschiene (20) sind die Direktionspunkt A (1), B (2) und der Induktionspunkt C (3) in Form von Bohrungen markiert. Auf der Unterkieferschiene (21) sind die Direktionspunkt A' (4), B' (5) und der Induktionspunkt C' (6) in Form von Bohrungen markiert. Figur 6C zeigt die Darstellung eines Intraoralscan eines Oberkiefers, der bei eingesetzter Oberkieferschiene (20) durchgeführt wurde. Der Induktionspunkt C (3) ist durch eine Bohrung in der Oberkieferschiene (20) markiert.

### Detaillierte Beschreibung der Erfindung

In einem ersten Aspekt betrifft die Erfindung ein Verfahren umfassend die Schritte
a) Bereitstellen einer ersten medizintechnischen Darstellung eines Kiefers mit Oberkiefer und Unterkiefer,
   a₁ Erfassen eines anatomisch stabilen rechten Direktionspunkts A, eines anatomisch stabilen linken Direktionspunkts B und eines anatomisch stabilen palatinal im Frontzahnbereich gelegenen Induktionspunkts C des Oberkiefers in der ersten Darstellung,
   a₂ Erfassen eines anatomisch stabilen rechten Direktionspunkts A', eines anatomisch stabilen linken Direktionspunkts B' und eines anatomisch stabilen labial gelegenen Induktionspunkts C' des Unterkiefers in der ersten Darstellung,
   a₃ Bilden eines ersten geometrischen Körpers mit einer ersten Fläche zwischen den Punkten A, B, und C' und einer zweiten Fläche zwischen den Punkten A', B' und C in der ersten Darstellung,
b) Bereitstellen einer zweiten medizintechnischen Darstellung des Kiefers,
   b₁ Erfassen eines anatomisch stabilen rechten Direktionspunkts A, eines anatomisch stabilen linken Direktionspunkts B und eines anatomisch stabilen palatinal im Frontzahnbereich gelegenen Induktionspunkts C des Oberkiefers in der zweiten Darstellung,
   b₂ Erfassen eines anatomisch stabilen rechten Direktionspunkts A', eines anatomisch stabilen linken Direktionspunkts B' und eines anatomisch stabilen labial gelegenen Induktionspunkts C' des Unterkiefers in der zweiten Darstellung,
   b₃ Bilden eines zweiten geometrischen Körpers mit einer ersten Fläche zwischen den Punkten A, B, und C' und einer zweiten Fläche zwischen den Punkten A', B' und C in der zweiten Darstellung, und
c) In Bezug setzen der ersten und zweiten Darstellung indem der erste geometrische Körper und der zweite geometrische Körper als gemeinsame Referenz dienen, wobei die Schritte a - c mit einem Computerprogramm durchgeführt werden.

Das erfindungsgemäße Verfahren ermöglicht einen Vergleich von zwei oder mehreren verschiedenen Darstellungen eines Kiefers unabhängig davon, ob die Aufnahmen unter denselben Bedingungen oder mit demselben Verfahren erstellt wurden. Dadurch kann die Differenz zwischen zwei unterschiedlichen Zuständen, die durch die erste und zweite Darstellung wiedergegeben sind, ermittelt werden. Auf Basis dieser Differenz kann anschließend die Gestalt eines zahntechnischen Erzeugnisses festgelegt und dieses gefertigt werden. Stellt eine der Darstellungen einen medizinisch vorteilhaften ursprünglichen Kieferzustand dar, von dem der aktuelle Zustand abweicht, so kann ein zahntechnisches Erzeugnis gefertigt werden, um diesen ursprünglichen Zustand zu rekonstruieren. Dadurch ist es möglich die ursprüngliche Artikulation und Okklusion des Kiefers wiederherzustellen und craniomandibuläre Dysfunktionen zu vermeiden.

Der Begriff "Darstellung" wie hier verwendet umfasst jede Wiedergabe eines Kiefers, wobei für das erfindungsgemäße Verfahren bevorzugt drei-dimensionale Darstellungen verwendet werden. Zu den möglichen Darstellungen zählen sowohl visuelle Wiedergaben, wie medizintechnische Aufnahmen, bevorzugt digitale drei-dimensionale Aufnahmen und Rekonstruktionen, als auch drei-dimensionale Modelle eines Kiefers oder Teilen davon, wie beispielsweise Gipsmodelle der Zahnbögen des Ober- und Unterkiefers.

Der Begriff "Kiefer" wie hier verwendet umfasst Ober- und Unterkiefer, wobei sowohl die Kieferknochen als auch das Gebiss bzw. die Zähne, sofern und soweit vorhanden, mit einschlossen sind.

Dem Verfahren liegt die Erkenntnis zugrunde, dass sich anhand von sechs anatomisch stabilen Referenzpunkten im Ober- und Unterkiefer (Direktionspunkte A, A', B, B' und Induktionspunkte C und C') ein geometrischer Körper bilden lässt. Dieser Körper ergibt sich aus einer ersten Fläche, die von den Punkten A, B und C' gebildet wird, und aus einer zweiten Fläche, die aus den Punkten A', B' und C gebildet wird. Indem die Referenzpunkte in jeder Darstellung des Kiefers identifiziert werden und aus ihnen für jede Darstellung ein geometrischer Körper gebildet wird, können die unterschiedlichen Darstellungen zueinander in Bezug gesetzt werden. Der geometrische Körper wird durch die sechs stabilen Referenzpunkte festgelegt und ist für jeden Patienten einzigartig. Folglich ist der geometrische Körper auch für jede Darstellung des Kiefers eines Patienten derselbe, so dass er als zuverlässige Referenz dienen kann. Beispielsweise können zwei digitale Wiedergaben desselben Kiefers anhand des in jeder Darstellung gebildeten geometrischen Körpers ausgerichtet werden, so dass die unterschiedlichen Darstellungen des Kiefers einander entsprechend angeordnet werden können. Dies kann in getrennten Darstellungen oder in einer kombinierten Abbildung beider Darstellungen, beispielsweise als Überblendung, erfolgen. Ebenso ist es möglich zwei Gipsmodelle eines Kiefers anhand der geometrischen Körper, beispielsweise durch Verwendung einer Ausrichtungsvorrichtung, in die beide Modelle eingespannt werden, übereinstimmend anzuordnen. Mit Hilfe einer Übertragungsvorrichtung mit Abgreifspitzen, wie beispielsweise in EP 12 188 176.7 (EP2719357) beschrieben, kann die Differenz zwischen den beiden Darstellungen ausgemessen werden.

Zur Ausführung des Verfahrens werden zunächst in allen Darstellungen des Kiefers der Patienten die Referenzpunkte ermittelt und der geometrische Körper gebildet. Dabei sollte zumindest eine der Darstellungen nach Möglichkeit einen vollständigen Ober- und Unterkiefer zeigen, wobei der Kiefer voll oder teilweise bezahnt oder vollständig zahnlos sein kann. In dem Ober- und Unterkiefer werden jeweils zwei Direktionspunkte (ein rechter Direktionspunkt A, A' und ein linker Direktionspunkt B, B') und ein palatinal im Frontzahnbereich (Oberkiefer) bzw. labial (Unterkiefer) gelegener Induktionspunkt C, C' erfasst. Als Direktionspunkte und Induktionspunkte werden anatomisch stabile Punkte innerhalb des Kiefers gewählt, wobei die Direktionspunkte A/A' und B/B' an einander entsprechenden Punkten der rechten und linken Ober- bzw. Unterkieferhälfte liegen. Die rechten Direktionspunkte des Ober- und Unterkiefers (A, A') liegen in der rechten Kieferhälfte und die linken Direktionspunkte (B, B') an der gegenüberliegenden Position der linken Kieferhälfte. Der Induktionspunkt ist hingegen im Frontzahnbereich lokalisiert, wobei er im Oberkiefer palatinal, d.h. hinter den Frontzähnen, bevorzugt hinter den Schneidezähnen, und im Unterkiefer labial, d.h. vor den Frontzähnen, bevorzugt vor den Schneidezähnen, liegt. Der Begriff "anatomisch stabil" bezeichnet hierbei Punkte, deren Position im Kiefer in der Zeit, die zwischen den Aufnahmen der unterschiedlichen Darstellungen vergangen ist, unverändert geblieben ist. Bevorzugt handelt es sich um Punkte deren Position zeitlebens, oder zumindest nach dem Vollenden der Entwicklung, unverändert erhalten bleibt.

In einer bevorzugten Ausführungsform werden die anatomisch stabilen Direktionspunkte (A, A', B, B') und die anatomisch stabilen Induktionspunkte (C, C') nach der Staub-Cranial-Methode bestimmt. Die von Karl-Heinz Staub identifizierten und beschriebenen cranialen Referenzpunkte können in jedem Kiefer identifiziert werden und bleiben nach Beendigung des Entwicklungsstadiums unveränderlich erhalten (Teubner & Marinello 2006; Lampropoulos 2003). Gemäß dem Verfahren nach Staub ist der rechte Direktionspunkt A der Punkt in der rechten Hälfte des Oberkiefers, an dem die Linie, die sich von der Kieferkammmitte zum Hamulus pterygoideus fortsetzt, ihre Richtung von konvex zu konkav ändert und der linke Direktionspunkt B der Punkt in der linken Hälfte des Oberkiefers, an dem die Linie, die sich von der Kieferkammmitte zum Hamulus pterygoideus fortsetzt ihre Richtung von konvex zu konkav ändert. Der Induktionspunkt C ist der Punkt des Oberkiefers, an dem die Medianachse des Oberkiefers die posteriore Umrisslinie der Papilla schneidet (Figur 4 A). Des Weiteren ist der rechte Direktionspunkt A' definiert als der Punkt in der rechten Hälfte des Unterkiefers, an dem die mesio-distale Verlaufskurve der Kammverbindungslinie am distalen Ende der Trigona retromolare ihre Richtung von konvex zu konkav ändert. Der linke Direktionspunkt B' ist dementsprechend der Punkt in der linken Hälfte des Unterkiefers, an dem die mesio-distale Verlaufskurve der Kammverbindungslinie am distalen Ende der Trigona retromolare ihre Richtung von konvex zu konkav ändert. Der Induktionspunkt C' des Unterkiefers ist jener Punkt, an dem die Medianachse des Unterkiefers die Conclusionslinie schneidet. Als Conclusionslinie wird die Grenzlinie zwischen beweglicher und unbeweglicher Schleimhaut vor den Schneidezähnen bezeichnet (Figur 4 B). Die Verwendung der cranialen Referenzpunkte nach Staub ist insbesondere bevorzugt, wenn die verschiedenen Darstellungen in einem deutlichen zeitlichen Anstand, beispielsweise von mindestens zwei Jahren, aufgenommen wurden, da diese Punkte zeitlebens unverändert erhalten bleiben.

Für einen Vergleich von Aufnahmen, die in geringem zeitlichem Abstand erstellt wurden, können aber auch andere Punkte in der rechten und linken Hälfte, sowie dem Frontzahnbereich des Ober- und Unterkiefers gewählt werden. Vorrausetzung hierfür ist lediglich, dass die Punkte in allen Darstellungen eindeutig bestimmbar sind.

Die sechs Referenzpunkte können auf unterschiedliche Weise erfasst werden. Sie können beispielsweise anhand ihrer anatomischen Lage in jeder Darstellung bestimmt und markiert werden.

In einer bevorzugten Ausführungsform wurden die Referenzpunkte beim Erstellen der ersten und/oder zweiten Darstellung markiert, indem die Darstellung unter Verwendung einer zahnreihenunabhängigen Schiene für den Oberkiefer und einer zahnreihenunabhängigen Scheine für den Unterkiefer erstellt wurde. Dazu wird die Lage der Referenzpunkte, beispielsweise an Hand eines Gipsmodells des Kiefers des Patenten, ermittelt. Anschließend werden zwei zahnreihenunabhängige Schienen, eine für den Oberkiefer und eine für den Unterkiefer, erstellt, auf denen die Referenzpunkte markiert werden (Figur 6A, B). Für Aufnahmen mit sichtbarem Licht, beispielsweise Intraoralscans, können Farbmarkierungen bzw. Löcher definierter Größe, deren Mitte die Umfänge der einzelnen Referenzpunkte darstellen, auf der Schiene angebracht werden (Figur 6 C). Für Röntgen- oder tomographische Aufnahmen kann die Position der Referenzpunkte mittels opaker Materialien an der Schiene markiert werden. In der Darstellung des Kiefers erscheinen diese Markierungen später als weiße Punkte. Die Schienen werden während der Aufnahme der ersten und/oder zweiten Darstellung in den Kiefer des Patenten eingesetzt. Auf diese Weise werden mit Hilfe der Markierungen auf den Schienen die Referenzpunkte unmittelbar in den Darstellungen des Kiefers angezeigt.

Die Lage der Referenzpunkte kann auch unter Verwendungen eines Gipsmodells entweder manuell oder mittels KUM (Kurvenmessungen mit unbegrenzten Möglichkeiten)-Graphiken bestimmt werden (Staub 2002).

In einer bevorzugten Ausführungsform umfasst die erste und/oder zweite Darstellung, einen vollständigen Ober- und Unterkiefer. Indem die Darstellung einen vollständigen Kiefer zeigt, können alle sechs Referenzpunkte eindeutig identifiziert werden. Zeigt eine der Darstellungen lediglich einen Teil oder mehrere Teile des Ober- und/oder Unterkiefers, so dass nicht alle Referenzpunkte unmittelbar sichtbar sind, können die fehlenden Referenzpunkte errechnet werden. Der geometrische Körper wird hierzu anhand einer Darstellung, z.B. einem dreidimensionalen Gipsmodell des Kiefers, in der alle sechs Referenzpunkte zu erkennen sind, ermittelt. Anschließend kann ausgehend von mindestens drei Referenzpunkten die Position der übrigen Punkte errechnet werden. Bevorzug liegt jeweils mindestens einer der Referenzpunkte im Ober- und Unterkiefer.

Sobald die Referenzpunkte bestimmt sind, wird der geometrische Körper mittels zwei Flächen gebildet. Die erste Fläche wird aufgespannt zwischen den Direktionspunkten des Oberkiefers (A, B) und dem Induktionspunkt des Unterkiefers (C'). Die zweite Fläche wird aufgespannt zwischen den Direktionspunkten des Unterkiefers (A', B') und dem Induktionspunkt des Oberkiefers (C). Dabei schneiden sich die beiden Ebenen an einer für jeden Patienten individuellen und konstanten Stelle. Die Referenzpunkte werden in jeder Darstellung erfasst und es wird für jede Darstellung ein geometrischer Körper gebildet. Da die Referenzpunkte und somit auch der geometrische Körper für jeden Patienten spezifisch sind, stimmen die geometrischen Körper der unterschiedlichen Darstellungen überein. Dadurch können die unterschiedlichen Darstellungen miteinander in Bezug gesetzt werden, wobei der geometrische Körper als Referenz dient. Dies ist beispielsweise möglich, indem jedem Punkt in der Darstellung eine Koordinate in Relation zu dem geometrischen Körper zugeordnet wird. Indem die Koordinaten in jeder Darstellung identisch zugeordnet werden, bezeichnen dieselben Koordinaten unterschiedlicher Darstellungen identische Punkte im Kiefer des Patienten. Dadurch ist es möglich, exakt zu ermitteln, welcher Punkt in der ersten Darstellung welchem Punkt in der zweiten Darstellung entspricht.

In einer bevorzugten Ausführungsform werden die erste und zweite Darstellung in Bezug gesetzt, indem der erste und zweite geometrische Körper übereinstimmend angeordnet werden. Werden die Darstellungen so ausgerichtet, dass die geometrischen Körper übereinstimmend angeordnet sind, sind damit auch die abgebildeten Kiefer identisch positioniert. Dies ermöglicht einen unmittelbaren Vergleich der Darstellungen, auf dessen Basis Abweichungen zwischen den unterschiedlichen Zuständen berechnet werden können. Handelt es sich bei den Darstellungen um digitale drei-dimensionale Aufnahmen, können diese am Bildschirm virtuell so gedreht werden, dass die geometrischen Körper übereinstimmend ausgerichtet sind. Die Unterschiede zwischen den Aufnahmen sind dadurch exakt zu erfassen. Sie können unter Verwendung entsprechender Software berechnet, und die gewonnenen Daten zur Fertigung eines zahntechnischen Erzeugnisses verwendet werden. Werden als Darstellungen Gipsmodelle eines Kiefers verwendet, können diese in einer Ausrichtungsvorrichtung eingespannt und anhand der geometrischen Körper identisch positioniert werden. Dies bietet sich vor allem zur manuellen Fertigung und Anpassung von zahntechnischen Erzeugnissen an.

In einer bevorzugten Ausführungsform werden die erste und zweite Darstellung in einer gemeinsamen Wiedergabe überblendet, wobei der erste und zweite geometrische Körper deckungsgleich angeordnet werden. Dazu werden mehrere dreidimensionale digitale Wiedergaben eines Kiefers in einer einzigen Abbildung überblendet, d.h. übereinander gelegt, so dass beide Darstellungen sichtbar bleiben, wodurch eine neue Wiedergabe des Kiefers geschaffen wird. Indem die geometrischen Körper in den Darstellungen deckungsgleich angeordnet werden, sind auch die Kiefer exakt deckungsgleich positioniert. Diese Ausführungsform ist insbesondere geeignet, um unterschiedliche Darstellungen eines Kiefers zu kombinieren, die mit unterschiedlichen Aufnahmetechniken erstellt wurden. Dadurch können die verschieden Informationen die durch unterschiedliche Aufnahmeverfahren gewonnen werden, in einer einzigen Darstellung vereint werden. Beispielsweise kann eine digitale Volumentomographieaufnahme und ein Intraoralscan in einer einzigen Abbildung überblendet werden. Bei der Fertigung eines zahntechnischen Erzeugnisses können so sowohl der anatomische Zustand des Kiefers als auch die Oberflächengegebenheiten der Zähne berücksichtigt werden. Darüber hinaus ist es auch möglich Aufnahmen, die zu verschiedenen Zeitpunkten erstellt wurden, zu überblenden.

In einer bevorzugten Ausführungsform umfasst das erfindungsgemäße Verfahren ferner den Schritt Bereitstellen mindestens einer weiteren medizintechnischen Darstellung des Kiefers und Durchführen der Schritte b₁ bis b₃ für die mindestens eine weitere Darstellung, wobei in Schritt c die erste, die zweite und mindestens eine weitere Darstellung in Bezug gesetzt werden, indem der erste, zweite und mindestens ein weiterer geometrischer Körper als gemeinsame Referenz dienen. Das Verfahren lässt sich mit beliebig vielen Darstellungen des Kiefers durchführen. Demgemäß lassen sich auch alle hierin beschriebenen Ausführungsformen des Verfahrens mit mehreren Darstellungen umsetzen. Beispielsweise können eine Vielzahl von Darstellungen, die über mehrere Jahre hinweg aufgenommen wurden, verglichen werden, um die Veränderungen des Kiefers über die Zeit zu ermitteln.

In einer bevorzugten Ausführungsform umfasst das erfindungsgemäße Verfahren ferner das Bilden mindestens einer Schnittebene durch die geometrischen Körper in der ersten und zweiten Darstellung, dadurch gekennzeichnet, dass die erste und zweite Darstellung zu einander in Bezug gesetzt werden, indem mindestens ein Teil der mindestens einen Schnittebene der ersten Darstellung und der entsprechende Teil der mindestens einen Schnittebene der zweiten Darstellung übereinstimmend angeordnet werden. Unter Verwendung der geometrischen Körper, die in jeder der Darstellungen gebildet werden und in ihrer Gestalt identisch sind, ist es möglich, Schnittebenen durch die Darstellungen zu legen, deren Positionen in Bezug auf den dargestellten Kiefer eindeutig definiert sind. Für Ausführungsformen des Verfahrens bei denen Schnittebenen gebildet werden, werden bevorzugt dreidimensionale digitale Darstellungen des Kiefers verwendet, beispielsweise drei-dimensionale Röntgenbilder, Volumentomographieaufnahmen und/oder Interaoralscans. Die Schnittebenen werden dabei nicht wie herkömmlich im Verhältnis zu Abmessungen der Darstellung bestimmt, sondern in Bezug auf den Kiefer des Patienten. Dies ist möglich, indem die Schnittebenen an dem geometrischen Körper ausgerichtet werden, der wiederum unmittelbar durch die Anatomie des Kiefers des Patienten bestimmt ist. Um die beiden Darstellungen des Kiefers zueinander in Bezug zu setzen, werden die Ebenen an identischen Positionen des geometrischen Körpers in der ersten und zweiten Darstellung aufgespannt. Der geometrische Körper, welcher für jede Darstellung des Kiefers eines Patienten identisch ist, dient dabei als Referenz, so dass einander exakt entsprechende Schnittebenen in unterschiedlichen Darstellungen gebildet werden können. Das Einziehen einzelner oder mehrerer Ebenen in die verschiedenen Darstellungen des Kiefers erlaubt einen Vergleich einzelner einander exakt entsprechender Teile des Kiefers in den unterschiedlichen Darstellungen. Dies kann anhand einzelner Darstellungen, die einander gegenübergestellt werden, erfolgen, wobei die Kiefer so dargestellt werden, dass die Schnittebenen übereinstimmend angeordnet sind. Alternativ dazu können die Teile der Darstellungen, die von den Schnittebenen erfasst werden, in einer gemeinsamen Wiedergabe überblendet werden, wobei die Schnittebenen deckungsgleich angeordnet werden. Mit Hilfe der Schnittebenen können auch einzelne Bereiche des Kiefers aus einer Darstellung extrahiert werden, um sie mit einer zweiten Darstellung an der ihnen entsprechenden Position zu überblenden. Ebenso können einzelne Ebenen einer Darstellung, beispielsweise jene Ebenen, die die Zahnkronen des Unterkiefers umfassen, in eine andere Darstellung hineinprojiziert werden. Dazu werden die Schnittebenen aus der ersten Darstellung an die entsprechende Position der Schnittebenen in der zweiten Darstellung projiziert, um diese zu ersetzen oder die vorhandenen zu überblenden. Werden auf diese Weise zwei Darstellungen analysiert, die zu unterschiedlichen Zeitpunkten im Leben eines Patienten aufgenommen wurden, können spezifische Veränderungen an bestimmten Positionen des Gebisses oder der Kieferknochen identifiziert und Abweichungen berechnet werden. Ein Zahnersatz oder eine zahnerhaltenden Maßnahme kann dann so ausgeführt werden, dass diese Veränderungen ausgeglichen werden und damit eine optimale Okklusion und Artikulation wieder hergestellt wird.

In einer bevorzugten Ausführungsform umfasst das erfindungsgemäße Verfahren ferner die Schritte
a₄ Bilden einer ersten vertikalen Schnittebene Sᵥ⁰ durch den geometrischen Körper in der ersten Darstellung, die durch die Punkte A, A', B und B' aufgespannt wird und Bilden mindestens einer weiteren vertikalen Schnittebene Svⁿ, die parallel zu Sᵥ⁰ angeordnet ist, und
b₄ Bilden einer ersten vertikalen Schnittebene Sᵥ⁰ durch den geometrischen Körper in der zweiten Darstellung, die durch die Punkte A, A', B und B' aufgespannt wird und Bilden mindestens einer weiteren vertikalen Schnittebene Svⁿ, die parallel zu Sᵥ⁰ angeordnet ist.

Die erste und zweite Darstellung werden dann zu einander in Bezug gesetzt, indem mindestens ein Teil mindestens einer der vertikalen Schnittebenen der ersten Darstellung und der entsprechende Teil der entsprechenden vertikalen Schnittebenen der zweiten Darstellung übereinstimmend angeordnet werden. Die Referenzpunkte A, A', B und B' definieren eine spezifische Ebene des geometrischen Körpers. Wie auch der geometrische Körper selbst, ist diese Ebene durch die anatomisch stabilen Punkte im Kiefer des Patienten festgelegt und somit für jeden Patienten individuell und beständig. Die durch die Punkte A, A', B und B' aufgespannte Ebene dient als Ausgangsebene Sᵥ⁰. Parallel zu dieser können weitere Schnittebenen (Sᵥ¹, Sᵥ², Sᵥ³, ... Sᵥⁿ) aufgespannt werden. Dabei werden in der ersten und zweiten Darstellung des Kiefers sich entsprechende Ebenen gebildet. Da der geometrische Körper, der für beide Darstellungen identisch ist, jeweils als Referenz dient, entsprechen sich die jeweiligen Ebenen und erfassen die sich entsprechenden Bereiche des Kiefers in den unterschiedlichen Darstellungen. Es können beliebig viele Ebenen in die Darstellungen eingezogen werden. Zur Analyse können einzelne Ebenen, Gruppen von Ebenen oder Teile einer oder mehrer Ebene extrahiert und einander gegenübergestellt werden. So können beispielsweise individuelle Bereiche mehrerer Ebenen, die gemeinsam einen einzelnen Zahn erfassen aus einer Darstellung extrahiert und zur Überblendung in eine zweite Darstellung importiert werden. In der zweiten Darstellung werden die importierten Ebenen bzw. Teile davon an der ihnen entsprechenden Position mit den entsprechenden Ebenen der zweiten Darstellung deckungsgleich angeordnet. Mit Hilfe einer Überblendung lassen sich so die Unterschiede zwischen der ersten und zweiten Darstellung des betroffenen Zahns unmittelbar visualisieren und Abweichungen berechnen. Dadurch ist es auch möglich, geringfügige Veränderungen im Gebiss oder im Kieferknochen des Patienten zu erkennen, die bei einem herkömmlichen statischen Vergleich unterschiedlicher Darstellung nicht erkennbar wären. Die Bildung vertikaler Schnittebenen ausgehend von der Ebene Sᵥ⁰ eignet sich vor allem für Anwendungen bei denen die Zähne oder Kieferknochen von anterior, posterior oder lateral betrachtet werden.

In einer bevorzugten Ausführungsform, umfasst das erfindungsgemäße Verfahren ferner die Schritte
a₅ Erfassen eines ersten Durchtrittspunkts (D₁) und eines zweiten Durchtrittspunkts (D₂) des geometrischen Körpers in der ersten Darstellung, wobei D₁ der rechte und D₂ der linke der Punkte ist, an denen die Kanten AC' und BC' der ersten Fläche die zweite Fläche durchqueren, die Kanten A'C und B'C der zweiten Fläche die erste Fläche durchqueren, oder die Kante AC' oder BC' der ersten Fläche die zweite Fläche durchquert und die Kante A'C oder B'C der zweiten Fläche die erste Fläche durchquert,
a₆ Bilden einer ersten horizontalen Schnittebene S_{H}⁰ in der ersten Darstellung, die durch die Punkte D₁, D₂ und einen Punkt E auf einer Linie durch die Induktionspunkte C und C' aufgespannt wird und Bilden mindestens einer weiteren Schnittebene S_{H}ⁿ, die parallel zu der ersten Schnittebene angeordnet ist,
b₅ Erfassen eines ersten Durchtrittspunkts (D₁) und eines zweiten Durchtrittspunkts (D₂) des geometrischen Körpers in der zweiten Darstellung, wobei D₁ der rechte und D₂ der linke der Punkte ist, an denen die Kanten AC' und BC' der ersten Fläche die zweite Fläche durchqueren, die Kanten A'C und B'C der zweiten Fläche die erste Fläche durchqueren, oder die Kante AC' oder BC' der ersten Fläche die zweite Fläche durchquert und die Kante A'C oder B'C der zweiten Fläche die erste Fläche durchquert, und
b₆ Bilden einer ersten horizontalen Schnittebene S_{H}⁰ in der zweiten Darstellung, die durch die Punkte D₁, D₂ und einen Punkt E auf einer Linie durch die Induktionspunkte C und C' aufgespannt wird und Bilden mindestens einer weiteren Schnittebene S_{H}ⁿ, die parallel zu der ersten Schnittebene angeordnet ist.

Die erste und zweite Darstellung werden zu einander in Bezug gesetzt, indem mindestens ein Teil mindestens einer der horizontalen Schnittebenen der ersten Darstellung und der entsprechende Teil der entsprechenden horizontalen Schnittebenen der zweiten Darstellung übereinstimmend angeordnet werden. Der geometrische Körper wird durch zwei Flächen gebildet, die zwischen den Punkten A, B und C' einerseits und A', B' und C andererseits aufgespannt werden. Indem bei der Bildung beider Flächen jeweils Punkte aus dem Ober- und Unterkiefer einbezogen werden, kreuzen sich die erste und zweite Fläche des geometrischen Körpers. Die Linie, auf der sich die erste und zweite Fläche kreuzen ist durch die anatomisch stabilen Punkte im Kiefer des Patienten definiert. Insgesamt können drei verschiedene Arten der Überkreuzung der ersten und zweiten Fläche des geometrischen Körpers beobachtet werden. Zum einen können D₁ der rechte und D₂ der linke der Punkte sein, an denen die Kanten AC' und BC' der ersten Fläche die zweite Fläche durchqueren oder die Kanten A'C und B'C der zweiten Fläche die erste Fläche durchqueren. Ein besonderer Fall dieser Konstellation liegt vor, wenn die Referenzpunkte A, A', B, B', C und C' weitgehend symmetrisch verteilt sind. Dann kreuzen sich die erste und zweite Fläche so, dass sich die Längsachsen [AC'] und [A'C] einerseits und [BC'] und [B'C] andererseits überschneiden (Figur 1 A). In diesem Fall liegen die Durchtrittspunkte D₁ und D₂ exakt an den Schnittpunkten der Längskanten [AC'] und [A'C] und [BC'] und [B'C]. Da die Kiefer der meisten Patienten jedoch nicht exakt symmetrisch sind, überkreuzen sich die erste und zweite Fläche des geometrischen Körpers in der Regel so, dass mindestens eine Längskante einer Fläche die andere Fläche durchtritt. Ist etwa die erste Fläche (A, B, C') schmäler als die zweite Fläche (A', B', C) durchtritt sie die zweite Fläche ohne dabei deren Längskanten zu tangieren (Figur 1 B). Ist die zweite Fläche (A', B', C) schmäler als die erste Fläche (A, B, C') durchkreuzt sie die erste Fläche, wobei beide Längskanten die erste Fläche in deren Inneren durchkreuzen (Figur 1 C). In einer dritten Konstellation durchquert die Kante AC' oder BC' der ersten Fläche die zweite Fläche und die Kante A'C oder B'C der zweiten Fläche die erste Fläche. Sind die erste und zweite Fläche des geometrischen Körpers aufgrund der Lage der Referenzpunkte nicht unmittelbar übereinander angeordnet, sondern zueinander verschoben, überkreuzen sich die beiden Flächen derart, dass jeweils eine Kante der einen Fläche durch die andere Fläche in deren Inneren hindurch tritt (Figur 1 D). In allen Fällen entsteht eine Schnittlinie zwischen jenen zwei Punkten, in denen die Längskante bzw. Längskanten der einen Fläche die andere Fläche durchkreuzen. Diese Punkte werden als Durchtrittspunkte (D₁, D₂) bezeichnet. Die Strecke zwischen den Punkten D₁ und D₂ ist für jeden Patienten individuell und beständig. Die Länge und Position der Linie kann genutzt werden, um eine definierte horizontale Schnittebene durch den geometrischen Körper zu legen. Neben den Punkten D₁ und D₂ wird die horizontale Ausgangsebene S_{H}⁰ durch einen weiteren Punkt E definiert. E ist ein beliebiger Punkt auf einer Linie durch die Induktionspunkte C und C', bevorzugt ein beliebiger Punkt auf einer Linie innerhalb der Induktionspunkte C und C' und besonders bevorzugt der Mittelpunkt der Linie CC'. Zwischen diesen Punkten (D₁, D₂ und E) wird eine Ebene in der ersten und zweiten Darstellung aufgespannt, die jeweils die Ebene S_{H}⁰ bildet und die sich in beiden Darstellungen an der exakt selben Position in Bezug auf den Kiefer des Patienten befindet. Oberhalb und unterhalb der Ausgangsebene S_{H}⁰ können beliebig viele weitere Ebenen parallel zu dieser eingezogen werden. Anhand der Ebenen können einzelne Bereiche des Kiefers in den unterschiedlichen Darstellungen verglichen oder überlagert werden. Es ist auch möglich einzelne Ebenen aus einer Darstellung zu extrahieren und in eine zweite Darstellung zu projizieren, in der sie deckungsgleich zu den ihnen entsprechenden Ebenen angeordnet werden. Anstatt vollständiger Ebenen können auch nur Teile einzelner oder mehrerer Ebenen, die beispielsweise einen einzelnen Zahn, eine Zahnreihe, einen Unterkiefer, einen Oberkiefer, die Oberfläche einer Zahnreihe, ein Implantat oder eine Krone erfassen zur weiteren Analyse aus einer Darstellungen extrahiert werden. Auch ist eine Überlagerung einzelner Teile einer ersten Darstellung mit einer weiteren Darstellung möglich, wobei die importierten Ebenen mit den ihnen entsprechenden Ebenen der weiteren Darstellungen deckungsgleich angeordnet werden. Die Erstellung horizontaler Schnittebenen ist insbesondere geeignet, wenn die Aufsicht auf einen Zahn oder eine Zahnreihe gewünscht ist.

In einer bevorzugten Ausführungsform umfasst das erfindungsgemäße Verfahren ferner das Bereitstellen mindestens einer weiteren Darstellung des Kiefers und Durchführen der Schritte b₁ bis b₄, bevorzugt der Schritte b₁ bis b₃, b₅ und b₆, ferner bevorzugt der Schritte b₁ bis b₆, für die mindestens eine weitere Darstellung, wobei in Schritt c mindestens ein Teil der ersten Darstellung mit der zweiten und mindestens einer weiteren Darstellung in Bezug gesetzt wird, indem mindestens ein Teil mindestens einer Schnittebene der ersten Darstellung und der entsprechende Teil der mindestens einen entsprechenden Schnittebene der zweiten und weiteren Darstellung deckungsgleich angeordnet werden. Indem mehrere Darstellungen, beispielsweise von unterschiedlichen Zeitpunkten, verwendet werden, ist es möglich, die Veränderung des Kiefers über die Zeit nachzuvollziehen. Ebenso kann eine erste Darstellung, die einen intakten Ursprungszustand wiedergibt, und eine zweite Darstellung, die den geschädigten Ist-Zustand weitergibt mit einer weiteren Darstellung eines geplanten Implantats kombiniert werden. Die Abmessungen des Implantats können dann so berechnet werden, dass es den Ursprungszustand wieder herstellt.

In einer bevorzugten Ausführungsform weist die erste, die zweite und/oder die weitere Darstellung neben dem Kiefer mindestens einen Teil einer knöchernen Struktur des Schädels, bevorzugt eines Schädelknochens, ferner bevorzugt ausgewählt aus der Gruppe bestehend auch Jochbein, Keilbein, Stirnbein, Schläfenbein und Scheitelbein, auf. Unter Verwendung des erfindungsgemäßen Verfahrens ist es möglich, zwei unterschiedliche Darstellungen des Kiefers eines Patienten unmittelbar und anatomisch zuverlässig zu vergleichen. Dabei können auch weitere Strukturen des Kopfbereichs miteinbezogen werden. So ist es möglich, Darstellungen, die einen Teil oder den gesamten Kopf eines Patienten erfassen durch Bilden des geometrischen Körpers anhand der anatomisch stabilen Referenzpunkte identisch auszurichten. Ebenso können, beispielsweise für eine Kieferrekonstruktion, ältere Darstellungen des Kiefers des Patienten mit aktuellen CT-Aufnahmen, die den kompletten Kopf erfassen, verglichen und in Bezug zueinander gesetzt werden. Dies erleichtert die Rekonstruktion des Kiefers um nach Möglichkeit den Ausgangszustand wiederherzustellen. Anwendungen dieses Verfahrens sind insbesondere vorteilhaft, in der Mund-, Kiefer- und Gesichtschirurgie, der Oralchirurgie und der Epithetik. Beispielsweise können Kieferfrakturen in allen Bereichen des Gebisssystems heutzutage mit Hilfe von Osteosyntheseschrauben und/oder durch eine intermaxiläre Fixierung versorgt werden. Bei Frakturen im Bereich des Kiefergelensköpfchens ist es dabei vorteilhaft die Osteosyntheseschrauben erst zu setzen, nachdem Ober- und Unterkiefer in einen Idealzustand gebracht werden konnten. Um diesen Zustand festzustellen und eine Schiene zur Fixierung des Ober- und Unterkiefers zu fertigen, kann der Idealzustand unter Verwendung des erfindungsgemäßen Verfahrens ermittelt werden. Dazu werden eine Darstellung, die den ursprünglichen Ausgangszustand wiedergibt und eine Darstellung, die den Ist-Zustand wiedergibt verglichen und anhand der so ermittelten Differenz zwischen Soll- und Ist-Zustand eine Schiene zur Fixierung von Ober- und Unterkiefer gefertigt. Unter Verwendung dieser Schiene während der Operation werden dann die Osteosyntheseschrauben gesetzt. Dadurch ist eine wesentliche exaktere Operation möglich, wodurch auch eine deutliche Verbesserung im Nachsorgeprozess des Patienten unter Vermeidung von Problemen bei Artikulation und Okklusion ermöglicht wird. Anstatt des vollständigen Schädels oder einzelner Schädelknochen können auch nur Teile davon in den Darstellungen wiedergegeben sein. Ebenso ist es möglich, nach der übereinstimmenden Ausrichtung der unterschiedlichen Darstellungen die Analyse auf einzelne Bereiche der Darstellung zu beschränken. Hierzu können unter Verwendung einzelner oder mehrerer Schnittebenen Teile von Darstellungen extrahiert werden.

In einer bevorzugten Ausführungsform wurden die erste und zweite Darstellung des Kiefers mit unterschiedlichen bildgebenden Verfahren erfasst. Das erfindungsgemäße Verfahren ist insbesondere geeignet um Darstellungen, die mit unterschiedlichen Techniken gewonnen wurden zu vergleichen bzw. zu überblenden. Häufig sind die Daten von Darstellungen, die mit unterschiedlichen bildgebenden Verfahren erstellt wurden, nicht kompatibel, so dass ein unmittelbarer Vergleich nicht möglich ist. Zudem liegen Aufnahmen, die den Ursprungszustand des Kiefers wiedergeben, häufig mehrere Jahre zurück, so dass sich allein aufgrund der technischen Weiterentwicklung Kompatibilitätsprobleme ergeben. Mit Hilfe des erfindungsgemäßen Verfahrens ist es dennoch möglich, diese unterschiedlichen Darstellungen übereinstimmend auszurichten, so dass jedenfalls ein unmittelbarer Vergleich anhand zwei getrennter Darstellungen, die jedoch übereinstimmend ausgerichtet sind, möglich ist. Darüber hinaus stellen Darstellungen unterschiedlicher Erfassungssysteme in der Regel unterschiedliche einander ergänzende Informationen zur Verfügung. So geben tomographische Aufnahmeverfahren die Anatomie der Kieferknochen und die Lage und Anordnung der Zähne in diesen wieder. Intraoralscans stellen demgegenüber Informationen über die Oberfläche des Kiefers und der Zähne zur Verfügung. Werden beide Darstellungen in einem einheitlichen Datenformat zur Verfügung gestellt (beispielsweise DICOM, digitale Bildverarbeitung und -kommunikation in der Medizin), können die Darstellungen zu einer einzigen Wiedergabe kombiniert werden, wobei eine übereinstimmende Ausrichtung anhand der geometrischen Körper in der ersten und zweiten Darstellung möglich ist. Besonders bevorzugt sind hierbei bildgebende Verfahren, die dreidimensionale Wiedergaben des Kiefers erstellen, vorzugsweise ausgewählt aus der Gruppe bestehend aus Computertomographie, digitale Volumentomographie, Magnetresonanztomographie, intraoraler Oberflächenscan und fotooptische Erfassungssysteme.

In einer bevorzugten Ausführungsform zeigen die erste und zweite Darstellung den Kiefer in mindestens zwei unterschiedlichen Zuständen. Mit dem erfindungsgemäßen Verfahren ist es möglich, Aufnahmen eines Kiefers, die zu unterschiedlichen Zeitpunkten angefertigt wurden, unmittelbar und exakt zu vergleichen. Beispielsweise können Aufnahmen, die während der Entwicklung eines Patienten gemacht wurden, verwendet werden, um die mit der Entwicklung einhergehenden Veränderungen des Kiefers zu ermitteln. Darstellungen, die zu unterschiedlichen Zeitpunkten im erwachsenen Stadium des Patienten gemacht wurden, können verwendet werden, um Abnutzungserscheinungen oder die Folgen craniomandibulärer Dysfunktionen (z.B. Zähneknirschen) zu eruieren. Durch den Vergleich unterschiedlicher Darstellungen können Veränderungen am Kiefer, wie beispielsweise Verschiebungen der Zähne, Absenkungen im Kieferknochenbereich oder auch Abnutzungen des Zahnschmelzes erkannt werden und das Ausmaß der Abweichungen berechnet werden. Das Verfahren kann aber auch verwendet werden, um Aufnahmen, die vor und nach einer Verletzung oder vor und nach einem operativen Eingriff erstellt wurden, zu vergleichen. Wird eine Darstellung, die nach einer schwerwiegenden Verletzung gemacht wurde, mit einer älteren Darstellung, die noch den intakten Kiefer zeigt, verglichen, kann das Ausmaß der Verletzung vollständig ermittelt werden. Dadurch ist es insbesondere auch möglich, Veränderungen am Kiefer, die infolge des Unfalls entstanden sind von bereits früher entstandenen Abweichungen zu unterscheiden. Eine Verwendung des erfindungsgemäßen Verfahrens ist insbesondere dann vorteilhaft, wenn die nach der Verletzung erstellten Aufnahmen nicht unter denselben Bedingungen gewonnen werden konnten, wie die früheren Darstellungen. Nach schweren Verletzungen können häufig keine digitalen Volumentomographieaufnahmen, wie sie in der herkömmlichen Zahnmedizin üblich sind, gemacht werden, da diese im Sitzen erfolgen. Dagegen werden in der Regel umfangreiche computertomographische Aufnahmen angefertigt. Mit Hilfe des erfindungsgemäßen Verfahrens ist es möglich, die so gewonnnen computertomographischen Aufnahmen des Kopf- und Kieferbereichs mit bereits bestehenden Aufnahmen der digitalen Volumentomographie zu vergleichen.

In einer bevorzugten Ausführungsform weist die zweite und/oder weitere Darstellung eine Abbildung eines zahntechnischen Erzeugnisses, bevorzugt ausgewählt aus der Gruppe bestehend aus Füllung, Krone, Inlay, Brücke und Implantat auf. Unter Verwendung des erfindungsgemäßen Verfahrens kann die Position und Lage eines zahntechnischen Erzeugnisses im Vergleich zu einer Ausgangssituation analysiert werden. Die Ausgangssituation kann beispielsweise eine Darstellung des intakten Kiefers des Patienten sein oder aber auch eine Darstellung des bereits beschädigten Kiefers, jedoch bevor das zahntechnische Erzeugnis eingesetzt wurde. Auch ist es möglich durch den Vergleich einer ersten Darstellung, die den ursprünglich intakten Kiefer zeigt, und einer zweiten Darstellung, die den späteren beschädigten Kiefer zeigt, ein zahntechnisches Erzeugnis zu entwerfen. Dazu kann in die kombinierte Wiedergabe der ersten und zweiten Darstellung die Abbildung des geplanten zahntechnischen Erzeugnisses eingefügt werden. Durch den unmittelbaren Vergleich zwischen dem ursprünglichen und dem beschädigten Zustand ist es möglich, das zahntechnische Erzeugnis so anzupassen, dass der ursprüngliche Ausgangszustand wiederhergestellt werden kann. Insbesondere können Form, Abmessung, Lage und Ausrichtung des geplanten zahntechnischen Erzeugnisses genau bestimmt werden.

In einer bevorzugten Ausführungsform umfasst das Verfahren ferner den Schritt
d) Fertigen eines zahntechnischen Erzeugnisses an Hand der in Bezug gesetzten ersten und zweiten Darstellung des Kiefers.

Basierend auf dem Vergleich unterschiedlicher Darstellungen des Kiefers eines Patienten unter Verwendung des erfindungsgemäßen Verfahrens, ist es möglich, einen Zahnersatz oder eine zahnerhaltende Maßnahmen exakt zu planen. Durch den unmittelbaren Vergleich unterschiedlicher Darstellungen werden die bestehenden Abweichungen im Kiefer bestimmt bzw. berechnet, so dass anhand der gewonnenen Daten ein zahntechnische Erzeugnis, bevorzugt ein Implantat, eine Brücke, eine Prothesen, eine Teilprothese, eine Füllung, ein Inlay, eine Krone oder eine Teilkrone, hergestellt werden kann. Das erfindungsgemäße Verfahren ist insbesondere geeignet, zur Planung von zahntechnischen Erzeugnissen, die durch moderne digitale Frästechniken oder 3D-Plotting hergestellt werden. Dabei werden die durch einen Vergleich des Ist-Zustands mit einem Soll-Zustand gewonnenen Informationen verwendet, um ein geeignetes zahntechnisches Erzeugnis auszuwählen und dessen dreidimensionale Gestalt zu bestimmen. Die entsprechenden Informationen werden dann an eine digitale Fräse übermittelt, welche das passgenaue Erzeugnis herstellt. Dementsprechend wird das zahntechnische Erzeugnis bevorzugt mittels einer computergesteuerten Fräsmaschiene gefertigt wird. Es ist allerdings auch möglich anhand der gewonnenen Informationen manuell eine Füllung oder auch einen Knochenaufbau durchzuführen.

In einer bevorzugten Ausführungsform, werden die Schritte a bis c des Verfahrens mit einem Computerprogramm durchgeführt. Dies erlaubt eine schnelle und einfache Visualisierung insbesondere von dreidimensionalen Darstellungen. Unter Verwendung des erfindungsgemäßen Verfahrens ist es möglich, unterschiedliche Darstellungen eines Kiefers identisch auszurichten, so dass sie leicht und zuverlässig verglichen werden können, wenn die Darstellungen nebeneinander angezeigt werden. Ebenso ist es möglich verschiedene Darstellungen in eine einzige Wiedergabe zu integrieren. Unter Verwendung des geometrischen Körpers können die Darstellungen deckungsgleich angeordnet werden, so dass die unterschiedlichen Darstellungen des Kiefers exakt überblendet werden können. Darüber hinaus kann das Verfahren mit anderen computergestützten Techniken wie zum Beispiel der digitalen Frästechnik und Programmen zum Design von Kronen, Inlays oder Implantaten kombiniert werden.

In einem weiteren Aspekt betrifft die Erfindung ein Computerprogramm, welches einen Programmcode aufweist, um einen Computer zu steuern, um die Schritte des erfindungsgemäßen Verfahrens auszuführen, wenn das Computerprogramm auf einem Computer ausgeführt wird. Das Computerprogramm kann auf einem geeigneten Medium gespeichert werden, wie beispielsweise einem optischen Speichermedium oder einem Festkörperspeicher, der zusammen mit oder als Teil anderer Hardware zur Verfügung gestellt wird. Das Computerprogramm kann aber auch auf andere Weise vertrieben werden, beispielweise über das Internet oder andere Telekommunikationssysteme.

In einem weiteren Aspekt betrifft die Erfindung eine medizintechnische Darstellungsvorrichtung für die Wiedergabe von mindestens zwei medizintechnischen Darstellungen eines Kiefers, mit einer Datenspeicherungsvorrichtung, die eine erste und eine zweite medizintechnische Darstellung des Kiefers speichert, einer Datenverarbeitungsvorrichtung, die einen ersten geometrischen Körper in der ersten Darstellung und eine zweiten geometrischen Körper in der zweiten Darstellung bildet, und einer Graphikverarbeitungsvorrichtung, die die erste und zweite Darstellung wiedergibt, wobei die erste und zweite Darstellung übereinstimmend angeordnet werden indem der erste geometrische Körper und der zweite geometrische Körper als gemeinsame Referenz dienen. Der erste und zweite geometrische Körper werden jeweils mit einer ersten Fläche zwischen einem anatomisch stabilen rechten Direktionspunkts A, einem anatomisch stabilen linken Direktionspunkts B und einem anatomisch stabilen palatinal im Frontzahnbereich gelegenen Induktionspunkts C des Oberkiefers und einer zweiten Fläche zwischen einem anatomisch stabilen rechten Direktionspunkts A', einem anatomisch stabilen linken Direktionspunkts B' und einem anatomisch stabilen labial gelegenen Induktionspunkts C' des Unterkiefers gebildet.

### Beispiele

### 1. Herstellen einer Teilkrone

### 1.1 Erfassen des Ausgangszustands

Nach Abschluss der Entwicklung des Patienten wird der Ausgangszustand des voll entwickelten Kiefers einschließlich Gebisssituation dokumentiert. Hierzu werden für den Patienten spezifische zahnreihenunabhängige Schienen (20, 21) hergestellt. Auf diesen Schienen werden die anatomisch stabilen cranialen Referenzpunkte nach Staub markiert. Dazu wird an der Position der Direktionspunkte A (1) und B (2) und an der Position des Induktionspunktes C (3) Löcher von einem Durchmesser von 2 mm in die Schiene (20) für den Oberkiefer gebohrt. Diese Löcher werden anschließend mit röntgenopakem Material gefüllt. In gleicher Weise werden die Direktionspunkte A' (4) und B' (5) sowie der Induktionspunkt C' (6) an der Schiene (21) des Unterkiefers markiert. Anschließend wird eine digitale Volumentomographieaufnahme des Patienten mit eingesetzter Ober- und Unterkieferschiene (20, 21) erstellt und archiviert. Aufgrund des röntgenopaken Materials sind die cranialen Referenzpunkte auf der digitalen Volumentomographieaufnahme als weiße Punkte deutlich sichtbar.

### 1.2 Erfassen eines aktuellen Zustands mit Zahnläsionen

Der Ist-Zustand des Kiefers des Patienten einschließlich der vorhandenen Zahnläsionen wird mittels eines Intraoralscans dokumentiert. Um die kranialen Referenzpunkte zu markieren, werden dem Patienten während des Intraoralscans eine zahnreihenunabhängigen Oberkiefer- und eine Unterkieferschiene (20, 21) eingesetzt, auf der die cranialen Referenzpunkte durch Bohrungen markiert sind. Die Intraoralscanaufnahme wird gespeichert und archiviert.

### 1.3 Vergleich des Ausgangszustands mit dem aktuellen Zustand

Für die digitale Volumentomographiedarstellung sowie für die Darstellung des Intraoralscans werden anhand der cranialen Referenzpunkte geometrische Körper gebildet. Anschließend werden die Darstellungen in einer gemeinsamen Wiedergabe überblendet und übereinstimmend ausgerichtet, indem die geometrischen Körper deckungsgleich angeordnet werden. Damit kommen die einander entsprechenden Stellen des Kiefers der beiden unterschiedlichen Darstellungen exakt übereinander zum Liegen. Durch eine Überblendung bleiben beide Darstellungen sichtbar, so dass die Läsionen an den Zähnen als Differenz zwischen den beiden Darstellungen eindeutig und unmittelbar erkennbar sind. Für den Bereich der Läsion wird eine Teilkrone geplant, die den Differenzbereich vollständig abdeckt und in ihrer Ausgestaltung den Ausgangszustand wiederherstellen soll. Die Abmessungen der Abweichungen zwischen dem aktuellen Zustand und dem Ausgangszustand werden an eine digitale Fräsmaschine übermittelt. Diese fertigt eine Keramikteilkrone, welche einerseits an den aktuellen Zustand der Gebisssituation exakt angepasst ist und andererseits, nach dem Einsetzen in das Gebiss des Patienten, den Ausgangszustand wiederherstellt. Damit wird nicht nur die Ausgangshöhe der betroffenen Zähne wiederhergestellt, sondern auch deren ursprüngliche Okklusion sowie die ursprüngliche Artikulation des Kiefers. Auf diese Weise bleibt die physiologische Kraftverteilung im Kiefer erhalten bzw. wird rekonstruiert, so dass craniomandibulären Dysfunktionen vorgebeugt wird.

### 2. Herstellen eines Implantats

### 2.1 Erfassen der Ausgangssituation

Die Ausgangssituation des Kiefers wird entsprechend Beispiel 1 erfasst.

### 2.2 Erfassen des aktuellen Zustands

Unter Verwendung einer zahnreihenunabhängigen Oberkiefer- und einer Unterkieferschiene (20, 21), in denen die kranialen Referenzpunkte nach Staub mit röntgenopakem Material markiert sind, wird eine Digitalvolumentomographieaufnahme des Kiefers des Patienten erstellt. Auf dieser Aufnahme ist der aktuelle Zustand des Kiefers einschließlich des Fehlens eines Zahns zu erkennen. Darüber hinaus enthält die Aufnahme Markierungen der sechs kranialen Referenzpunkte.

### 2.3 Vergleich der Darstellungen und Herstellung des Implantats

In jeder der beiden Darstellungen (Ausgangszustand und Ist Zustand) wird ein geometrischer Körper anhand der sechs cranialen Referenzpunkte gebildet. Anschließend wird durch jeden der beiden geometrischen Körper eine horizontale Ausgangsebene (S_{H}⁰) gelegt, die durch die Durchtrittspunkte D₁ und D₂ und den Punkt E als Mittelpunkt der Linie CC' gespannt wird. Anschließend werden parallel zur Ausgangsebene weitere Ebenen eingeführt, die im Abstand von 1 mm den Bereich der Zahnlücke und des angrenzenden Gebissbereichs abdecken. Aus der ersten Darstellung werden die Ebenen im Bereich der Zahnlücke und des umgebenen Gebisses extrahiert und in die zweite Darstellung projiziert, wobei die Ebenen der ersten Darstellung deckungsgleich mit den ihnen entsprechenden Ebenen der zweiten Darstellung angeordnet werden. Dadurch wird die Position des fehlenden Zahnes entsprechend seiner Ausgangssituation im nunmehr beschädigten Gebiss visualisiert.

Anhand dieses Vergleichs wird ein Implantat, welches einerseits die Ausgangssituation des fehlenden Zahnes rekonstruieren soll und andererseits die nunmehr veränderte Umgebung (Ist-Zustand) des fehlenden Zahns berücksichtigt, entworfen und dessen Abmessungen berechnet. Anhand der so gewonnenen Informationen wird das Zahnimplantat gefertigt.

### Bezugszeichenliste

- 1: Direktionspunkt A
- 2: Direktionspunkt B
- 3: Induktionspunkt C
- 4: Direktionspunkt A'
- 5: Direktionspunkt B'
- 6: Induktionspunkt C'
- 7: erste Fläche
- 8: zweite Fläche
- 9: Außenkante [AC']
- 10: Außenkante [BC']
- 11: Außenkanten [A'C]
- 12: Außenkanten [B'C]
- 13: Durchtrittspunkte D₁
- 14: Durchtrittspunkte D₂
- 15: Schnittebene S_{V}⁰
- 16: Schnittebene S_{V}¹
- 17: Schnittebene S_{H}⁰
- 18: Schnittebene S_{H}¹
- 19: Papilla
- 20: Oberkieferschiene
- 21: Unterkieferschiene

### Referenzen

Teubner und Marinello, Die Berechnung der prospektiven Zahnposition anhand einer Modellanalyse - das Staub™-Cranial-System; Schweiz. Monatsschrift für Zahnmedizin Vol. 116; 7/2006;
Lampropoulos P, Das Staub™-Cranial-System - Reliabilität der Messpunkte zur Rekonstruktion der Zahnstellung im zahnlosen Kiefer; Inaugural-Dissertation Albert-Ludwigs-Universität Freiburg im Breisgau; 2003

## Patentansprüche

1. Verfahren umfassend die Schritte
a) Bereitstellen einer ersten medizintechnischen Darstellung eines Kiefers mit Oberkiefer und Unterkiefer,
a₁ Erfassen eines anatomisch stabilen rechten Direktionspunkts A, eines anatomisch stabilen linken Direktionspunkts B und eines anatomisch stabilen palatinal im Frontzahnbereich gelegenen Induktionspunkts C des Oberkiefers in der ersten Darstellung,
a₂ Erfassen eines anatomisch stabilen rechten Direktionspunkts A', eines anatomisch stabilen linken Direktionspunkts B' und eines anatomisch stabilen labial gelegenen Induktionspunkts C' des Unterkiefers in der ersten Darstellung,
a₃ Bilden eines ersten geometrischen Körpers mit einer ersten Fläche zwischen den Punkten A, B, und C' und einer zweiten Fläche zwischen den Punkten A', B' und C in der ersten Darstellung,
b) Bereitstellen einer zweiten medizintechnischen Darstellung des Kiefers,
b₁ Erfassen eines anatomisch stabilen rechten Direktionspunkts A, eines anatomisch stabilen linken Direktionspunkts B und eines anatomisch stabilen palatinal im Frontzahnbereich gelegenen Induktionspunkts C des Oberkiefers in der zweiten Darstellung,
b₂ Erfassen eines anatomisch stabilen rechten Direktionspunkts A', eines anatomisch stabilen linken Direktionspunkts B' und eines anatomisch stabilen labial gelegenen Induktionspunkts C' des Unterkiefers in der zweiten Darstellung,
b₃ Bilden eines zweiten geometrischen Körpers mit einer ersten Fläche zwischen den Punkten A, B, und C' und einer zweiten Fläche zwischen den Punkten A', B' und C in der zweiten Darstellung, und
c) In Bezug setzen der ersten und zweiten Darstellung indem der erste geometrische Körper und der zweite geometrische Körper als gemeinsame Referenz dienen, wobei die Schritte a - c mit einem Computerprogramm durchgeführt werden.

2. Verfahren nach Anspruch 1, **dadurch gekennzeichnet, dass** die anatomisch stabilen Direktionspunkte A, A', B, und B' und die anatomisch stabilen Induktionspunkte C und C' nach der Staub-Cranial Methode bestimmt werden.

3. Verfahren nach einem der Ansprüche 1 und 2, **dadurch gekennzeichnet, dass** die Referenzpunkte beim Erstellen der ersten und/oder zweiten Darstellung markiert wurden, indem die Darstellung unter Verwendung einer zahnreihenunabhängigen Schiene für den Oberkiefer und einer zahnreihenunabhängigen Scheine für den Unterkiefer erstellt wurde.

4. Verfahren nach einem der vorhergehenden Ansprüche, **dadurch gekennzeichnet dass** die erste und zweite Darstellung in Bezug gesetzt werden, indem der erste und zweite geometrische Körper übereinstimmend angeordnet werden.

5. Verfahren nach einem der vorhergehenden Ansprüche, **dadurch gekennzeichnet, dass** die erste und zweite Darstellung in einer gemeinsamen Wiedergabe überblendet werden, wobei der erste und zweite geometrische Körper deckungsgleich angeordnet werden.

6. Verfahren nach einem der vorhergehenden Ansprüche, ferner umfassend Bereitstellen mindestens einer weiteren medizintechnischen Darstellung des Kiefers und Durchführen der Schritte b₁ bis b₃ für die mindestens eine weitere Darstellung, wobei in Schritt c die erste, die zweite und mindestens eine weitere Darstellung in Bezug gesetzt werden, indem der erste, der zweite und mindestens ein weiterer geometrischer Körper als gemeinsame Referenz dienen.

7. Verfahren nach einem der vorhergehenden Ansprüche, ferner umfassend Bilden mindestens einer Schnittebene durch die geometrischen Körper in der ersten und zweiten Darstellung, **dadurch gekennzeichnet, dass** die erste und zweite Darstellung zu einander in Bezug gesetzt werden, indem mindestens ein Teil der mindestens einen Schnittebene der ersten Darstellung und der entsprechende Teil der mindestens einen Schnittebene der zweiten Darstellung übereinstimmend angeordnet werden.

8. Verfahren nach einem der vorhergehenden Ansprüche, ferner umfassend die Schritte
a₄ Bilden einer ersten vertikalen Schnittebene Sᵥ⁰ durch den geometrischen Körper in der ersten Darstellung, die durch die Punkte A, A', B und B' aufgespannt wird und Bilden mindestens einer weiteren vertikalen Schnittebene Sᵥⁿ, die parallel zu Sᵥ⁰ angeordnet ist, und
b₄ Bilden einer ersten vertikalen Schnittebene Sᵥ⁰ durch den geometrischen Körper in der zweiten Darstellung, die durch die Punkte A, A', B und B' aufgespannt wird und Bilden mindestens einer weiteren vertikalen Schnittebene Sᵥⁿ, die parallel zu Sᵥ⁰ angeordnet ist,
**dadurch gekennzeichnet, dass** die erste und zweite Darstellung zu einander in Bezug gesetzt werden, indem mindestens ein Teil mindestens einer der vertikalen Schnittebenen der ersten Darstellung und der entsprechende Teil der entsprechenden vertikalen Schnittebenen der zweiten Darstellung übereinstimmend angeordnet werden.

9. Verfahren nach einem der vorhergehenden Ansprüche, ferner umfassend die Schritte
a₅ Erfassen eines ersten Durchtrittspunkts (D₁) und eines zweiten Durchtrittspunkts (D₂) des geometrischen Körpers in der ersten Darstellung, wobei D₁ der rechte und D₂ der linke der Punkte ist, an denen die Kanten AC' und BC' der ersten Fläche die zweite Fläche durchqueren, die Kanten A'C und B'C der zweiten Fläche die erste Fläche durchqueren, oder die Kante AC' oder BC' der ersten Fläche die zweite Fläche durchquert und die Kante A'C oder B'C der zweiten Fläche die erste Fläche durchquert,
a₆ Bilden einer ersten horizontalen Schnittebene S_{H}⁰ in der ersten Darstellung, die durch die Punkte D₁, D₂ und einen Punkt E auf einer Linie durch die Induktionspunkte C und C' aufgespannt wird und Bilden mindestens einer weiteren Schnittebene S_{H}ⁿ, die parallel zu der ersten Schnittebene angeordnet ist,
b₅ Erfassen eines ersten Durchtrittspunkts (D₁) und eines zweiten Durchtrittspunkts (D₂) des geometrischen Körpers in der zweiten Darstellung, wobei D₁ der rechte und D₂ der linke der Punkte ist, an denen die Kanten AC' und BC' der ersten Fläche die zweite Fläche durchqueren, die Kanten A'C und B'C der zweiten Fläche die erste Fläche durchqueren, oder die Kante AC' oder BC' der ersten Fläche die zweite Fläche durchquert und die Kante A'C oder B'C der zweiten Fläche die erste Fläche durchquert, und
b₆ Bilden einer ersten horizontalen Schnittebene S_{H}⁰ in der zweiten Darstellung, die durch die Punkte D₁, D₂ und einen Punkt E auf einer Linie durch die Induktionspunkte C und C' aufgespannt wird und Bilden mindestens einer weiteren Schnittebene S_{H}ⁿ, die parallel zu der ersten Schnittebene angeordnet ist,
**dadurch gekennzeichnet, dass** die erste und zweite Darstellung zu einander in Bezug gesetzt werden, indem mindestens ein Teil mindestens einer der horizontalen Schnittebenen der ersten Darstellung und der entsprechende Teil der entsprechenden horizontalen Schnittebenen der zweiten Darstellung übereinstimmend angeordnet werden.

10. Verfahren nach einem der vorhergehenden Ansprüche, **dadurch gekennzeichnet, dass** die erste und zweite Darstellung des Kiefers mit unterschiedlichen bildgebenden Verfahren erfasst wurden.

11. Verfahren nach einem der vorhergehenden Ansprüche, **dadurch gekennzeichnet, dass** die erste und zweite Darstellung den Kiefer in mindestens zwei unterschiedlichen Zuständen zeigen.

12. Verfahren nach Anspruch 11, ferner umfassend den Schritt
d Fertigen eines zahntechnischen Erzeugnisses an Hand der in Bezug gesetzten ersten und zweiten Darstellung des Kiefers.

13. Computerprogramm aufweisend einen Programmcode um einen Computer zu steuern, um die Schritte des Verfahrens gemäß einem der Ansprüche 1 bis 12 auszuführen, wenn das Computerprogramm auf einem Computer ausgeführt wird.

14. Medizintechnische Darstellungsvorrichtung für die Wiedergabe von mindestens zwei medizintechnischen Darstellungen eines Kiefers, mit
- einer Datenspeicherungsvorrichtung, die eine erste und eine zweite medizintechnische Darstellung des Kiefers speichert,
- einer Datenverarbeitungsvorrichtung, die einen ersten geometrischen Körper in der ersten Darstellung und eine zweiten geometrischen Körper in der zweiten Darstellung bildet, und
- einer Graphikverarbeitungsvorrichtung, die die erste und zweite Darstellung wiedergibt, wobei die erste und zweite Darstellung übereinstimmend angeordnet werden indem der erste geometrische Körper und der zweite geometrische Körper als gemeinsame Referenz dienen,
**dadurch gekennzeichnet, dass** der erste und zweite geometrische Körper jeweils gebildet werden mit einer ersten Fläche zwischen einem anatomisch stabilen rechten Direktionspunkts A, einem anatomisch stabilen linken Direktionspunkts B und einem anatomisch stabilen palatinal im Frontzahnbereich gelegenen Induktionspunkts C des Oberkiefers und einer zweiten Fläche zwischen einem anatomisch stabilen rechten Direktionspunkts A', einem anatomisch stabilen linken Direktionspunkts B' und einem anatomisch stabilen labial gelegenen Induktionspunkts C' des Unterkiefers.

## Claims

1. A method, comprising the steps of
a) providing a first medical-technical representation of a jaw, with upper jaw and lower jaw,
a₁ recording an anatomically stable right direction point A, an anatomically stable left direction point B, and an anatomically stable induction point C in a palatinal position in the anterior tooth region of the upper jaw in the first representation,
a₂ recording an anatomically stable right direction point A', an anatomically stable left direction point B', and an anatomically stable induction point C' in a labial position of the lower jaw in the first representation,
a₃ forming a first geometric body with a first area between the points A, B and C' and a second area between the points A', B' and C in the first representation,
b) providing a second medical-technical representation of the jaw,
b₁ recording an anatomically stable right direction point A, an anatomically stable left direction point B and an anatomically stable induction point C in a palatinal position in the anterior tooth region of the upper jaw in the second representation,
b₂ recording an anatomically stable right direction point A', an anatomically stable left direction point B' and an anatomically stable induction point C' in a labial position of the lower jaw in the second representation,
b₃ forming a second geometric body with a first area between the points A, B and C' and a second area between the points A', B' and C in the second representation, and
c) setting in relation to each other the first and second representation, whereby the first geometric body and the second geometric body act as a joint reference, wherein the steps a - c are performed using a computer program.

2. The method according to claim 1, **characterized in that** the anatomically stable direction points A, A', B and B' and the anatomically stable induction points C and C' are determined according to the Staub Cranial method.

3. The method according to any of claims 1 and 2, **characterized in that** the reference points were marked when the first and/or second representation were/was created, whereby the representation was created using a teeth row-independent splint for the upper jaw and a teeth row-independent splint for the lower jaw.

4. The method according to any of the preceding claims, **characterized in that** the first and second representation are set in relation to each other by arranging the first and second geometric body to correspond to each other.

5. The method according to any of the preceding claims, **characterized in that** the first and second representation are superimposed in a joint display, wherein the first and second geometric body are arranged congruently.

6. The method according to any of the preceding claims, further comprising providing at least one further medical-technical representation of the jaw and performing the steps b₁ to b₃ for the at least one further representation, wherein, in step c, the first, the second and at least one further representation are set in relation to each other, whereby the first, the second and at least one further geometric body act as a joint reference.

7. The method according to any of the preceding claims, further comprising forming at least one sectional plane through the geometric bodies in the first and second representation, **characterized in that** the first and second representation are set in relation to each other by arranging at least one part of the at least one sectional plane of the first representation and the corresponding part of the at least one sectional plane of the second representation to correspond to each other.

8. The method according to any of the preceding claims, further comprising the steps of
a₄ forming a first vertical sectional plane Sᵥ⁰ through the geometric body in the first representation which is defined by the points A, A', B and B', and forming at least one further vertical sectional plane Sᵥⁿ, which is arranged parallel to Sᵥ⁰, and
b₄ forming a first vertical sectional plane Sᵥ⁰ through the geometric body in the second representation which is defined by the points A, A', B and B', and forming at least one further vertical sectional plane Sᵥⁿ, which is arranged parallel to Sᵥ⁰,
**characterized in that** the first and second representation are set in relation to each other by arranging at least one part of at least one of the vertical sectional planes of the first representation and the corresponding part of the corresponding vertical sectional planes of the second representation to correspond to each other.

9. The method according to any of the preceding claims, further comprising the steps of
a₅ recording a first penetration point (D₁) and a second penetration point (D₂) of the geometric body in the first representation, wherein D₁ is the right one and D₂ is the left one of the points where the edges AC' and BC' of the first area traverse the second area, the edges A'C and B'C of the second area traverse the first area, or the edge AC' or BC' of the first area traverses the second area and the edge A'C or B'C of the second area traverses the first area,
a₆ forming a first horizontal sectional plane S_{H}⁰ in the first representation which is defined by the points D₁, D₂ and a point E on a line through the induction points C and C', and forming at least one further sectional plane S_{H}ⁿ, which is arranged parallel to the first sectional plane,
b₅ recording a first penetration point (D₁) and a second penetration point (D₂) of the geometric body in the second representation, wherein D₁ is the right one and D₂ is the left one of the points where the edges AC' and BC' of the first are traverse the second area, the edges A'C and B'C of the second area traverse the first area, or the edge AC' or BC' of the first area traverses the second area and the edge A'C or B'C of the second area traverses the first area, and
b₆ forming a first horizontal sectional plane S_{H}⁰ in the second representation which is defined by the points D₁, D₂ and a point E on a line through the induction points C and C', and forming at least one further sectional plane S_{H}ⁿ, which is arranged parallel to the first sectional plane,
**characterized in that** the first and second representation are set in relation to each other by arranging at least one part of at least one of the horizontal sectional planes in the first representation and the corresponding part of the corresponding horizontal sectional planes of the second representation to correspond to each other.

10. The method according to any of the preceding claims, **characterized in that** the first and second representation of the jaw have been recorded using different imaging methods.

11. The method according to any of the preceding claims, **characterized in that** the first and second representation show the jaw in at least two different states.

12. The method according to claim 11, further comprising the step of
d manufacturing a dental product on the basis of the first and second representation of the jaw which have been set in relation to each other.

13. A computer program, comprising a program code for controlling a computer in order to perform the steps of the method according to any of claims 1 to 12 when the computer program is executed on a computer.

14. A medical-technical representation device for displaying at least two medical-technical representations of a jaw, comprising
- a data storage device which stores a first and a second medical-technical representation of the jaw,
- a data processing device which forms a first geometric body in the first representation and a second geometric body in the second representation, and
- a graphic processing device which displays the first and second representation, wherein the first and second representation are arranged to correspond to each other, whereby the first geometric body and the second geometric body act as a joint reference,
**characterized in that** the first and second geometric body are formed with a first area between an anatomically stable right direction point A, an anatomically stable left direction point B and an anatomically stable induction point C in a palatinal position in the anterior tooth region of the upper jaw, and with a second area between an anatomically stable right direction point A', an anatomically stable left direction point B' and an anatomically stable induction point C' in a labial position of the lower jaw, respectively.

## Revendications

1. Procédé, comprenant les étapes suivantes :
a) fournir une première représentation médico-technique d'une mâchoire, avec maxillaire supérieur et maxillaire inférieur,
a₁ enregistrer un point directionnel A de droite stable anatomiquement, un point directionnel B de gauche stable anatomiquement, et un point d'induction C stable anatomiquement, situé dans le secteur dentaire antérieur en position palatine, du maxillaire supérieur dans la première représentation,
a₂ enregistrer un point directionnel A' de droite stable anatomiquement, un point directionnel B' de gauche stable anatomiquement, et un point d'induction C' stable anatomiquement, situé en position labiale, du maxillaire inférieur dans la première représentation,
a₃ former un premier corps géométrique ayant une première surface entre les points A, B et C' et une seconde surface entre les points A', B' et C dans la première représentation,
b) fournir une deuxième représentation médico-technique de la mâchoire,
b₁ enregistrer un point directionnel A de droite stable anatomiquement, un point directionnel B de gauche stable anatomiquement, et un point d'induction C stable anatomiquement, situé dans le secteur dentaire antérieur en position palatine, du maxillaire supérieur dans la deuxième représentation,
b₂ enregistrer un point directionnel A' de droite stable anatomiquement, un point directionnel B' de gauche stable anatomiquement, et un point d'induction C' stable anatomiquement, situé en position labiale, du maxillaire inférieur dans la deuxième représentation,
b₃ former un deuxième corps géométrique ayant une première surface entre les points A, B et C', et une seconde surface entre les points A', B' et C, dans la deuxième représentation, et
c) mettre en relation la première et deuxième représentations, le premier corps géométrique et le deuxième corps géométrique servant de référence commune, les étapes a - c étant exécutées à l'aide d'un programme informatique.

2. Procédé selon la revendication 1, **caractérisé en ce que** les points directionnels A, A', B et B' stables anatomiquement et les points d'induction C et C' stables anatomiquement sont déterminés selon la méthode Staub Cranial.

3. Procédé selon l'une des revendications 1 et 2, **caractérisé en ce que** les points de référence ont été marqués lors de l'établissement de la première et/ou deuxième représentation/s en établissant la représentation en utilisant une gouttière indépendante de la rangée de dents pour le maxillaire supérieur et une gouttière indépendante de la rangée de dents pour le maxillaire inférieur.

4. Procédé selon l'une des revendications précédentes, **caractérisé en ce que** la première et deuxième représentations sont mises en relation l'une avec l'autre en disposant le premier et deuxième corps géométriques de manière à se correspondre.

5. Procédé selon l'une des revendications précédentes, **caractérisé en ce que** la première et deuxième représentations sont superposées dans un affichage conjoint, le premier et deuxième corps géométriques étant disposés de manière à coïncider.

6. Procédé selon l'une des revendications précédentes, comprenant en outre la fourniture d'au moins une autre représentation médico-technique de la mâchoire, et l'exécution des étapes b₁ à b₃ pour au moins une autre représentation, la première, la deuxième et au moins une autre représentations étant mises en relation l'une avec l'autre dans l'étape c, le premier, le deuxième et au moins un autre corps géométriques servant de référence commune.

7. Procédé selon l'une des revendications précédentes, comprenant en outre la formation d'au moins un plan de coupe à travers les corps géométriques dans la première et deuxième représentations, **caractérisé en ce que** la première et deuxième représentations sont mises en relation l'une avec l'autre en disposant au moins une partie de l'au moins un plan de coupe de la première représentation et la partie correspondante de l'au moins un plan de coupe de la deuxième représentation de manière à se correspondre.

8. Procédé selon l'une des revendications précédentes, comprenant en outre les étapes suivantes :
a₄ former un premier plan de coupe Sᵥ⁰ vertical à travers le corps géométrique dans la première représentation défini par les points A, A', B et B', et former au moins un autre plan de coupe Sᵥⁿ vertical disposé parallèlement à Sᵥ⁰, et
b₄ former un premier plan de coupe Sᵥ⁰ vertical à travers le corps géométrique dans la deuxième représentation défini par les points A, A', B et B', et former au moins un autre plan de coupe Sᵥⁿ vertical disposé parallèlement à Sᵥ⁰,
**caractérisé en ce que** la première et deuxième représentations sont mises en relation l'une avec l'autre en disposant au moins une partie de l'au moins un des plans de coupe verticaux de la première représentation et la partie correspondante des plans de coupe verticaux correspondants de la deuxième représentation de manière à se correspondre.

9. Procédé selon l'une des revendications précédentes, comprenant en outre les étapes suivantes :
a₅ enregistrer un premier point de passage (D₁) et un second point de passage (D₂) du corps géométrique dans la première représentation, D₁ étant le point de droite et D₂ étant le point de gauche des points où les bords AC' et BC' de la première surface traversent la seconde surface, où les bords A'C et B'C de la seconde surface traversent la première surface, ou où le bord AC' ou BC' de la première surface traverse la seconde surface et le bord A'C ou B'C de la seconde surface traverse la première surface,
a₆ former un premier plan de coupe S_{H}⁰ horizontal dans la première représentation défini par les points D₁, D₂ et un point E sur une ligne à travers les points d'induction C et C', et former au moins un autre plan de coupe S_{H}ⁿ disposé parallèlement au premier plan de coupe,
b₅ enregistrer un premier point de passage (D₁) et un second point de passage (D₂) du corps géométrique dans la deuxième représentation, D₁ étant le point de droite et D₂ étant le point de gauche des points où les bords AC' et BC' de la première surface traversent la seconde surface, où les bords A'C et B'C de la seconde surface traversent la première surface, ou où le bord AC' ou BC' de la première surface traverse la seconde surface et le bord A'C ou B'C de la seconde surface traverse la première surface, et
b₆ former un premier plan de coupe S_{H}⁰ horizontal dans la deuxième représentation défini par les points D₁, D₂ et un point E sur une ligne à travers les points d'induction C et C', et former au moins un autre plan de coupe S_{H}ⁿ disposé parallèlement au premier plan de coupe,
**caractérisé en ce que** la première et deuxième représentations sont mises en relation l'une avec l'autre en disposant au moins une partie de l'au moins un des plans de coupe horizontaux de la première représentation et la partie correspondante des plans de coupe horizontaux correspondants de la deuxième représentation de manière à se correspondre.

10. Procédé selon l'une des revendications précédentes, **caractérisé en ce que** la première et deuxième représentations de la mâchoire ont été enregistrées par des procédés d'imagerie différents.

11. Procédé selon l'une des revendications précédentes, **caractérisé en ce que** la première et deuxième représentations montrent la mâchoire dans au moins deux états différents.

12. Procédé selon la revendication 11, comprenant en outre l'étape suivante :
d produire un produit dentaire à l'aide de la première et deuxième représentations de la mâchoire qui ont été mises en relation l'une avec l'autre.

13. Programme informatique, comprenant un code de programme destiné à commander un ordinateur afin d'exécuter les étapes du procédé selon l'une des revendications 1 à 12 lorsque le programme informatique est exécuté sur un ordinateur.

14. Dispositif de représentation médico-technique conçu pour l'affichage d'au moins deux représentations médico-techniques d'une mâchoire, comprenant
- un dispositif de stockage de données qui stocke une première et une deuxième représentations médico-techniques de la mâchoire,
- un dispositif de traitement de données qui forme un premier corps géométrique dans la première représentation et un deuxième corps géométrique dans la deuxième représentation, et
- un dispositif de traitement graphique qui affiche la première et deuxième représentations, la première et deuxième représentations étant disposées de manière à se correspondre, le premier corps géométrique et le deuxième corps géométrique servant de référence commune,
**caractérisé en ce que** le premier et deuxième corps géométriques sont formés, respectivement, ayant une première surface entre un point directionnel A de droite stable anatomiquement, un point directionnel B de gauche stable anatomiquement, et un point d'induction C stable anatomiquement, situé dans le secteur dentaire antérieur en position palatine, du maxillaire supérieur et une seconde surface entre un point directionnel A' de droite stable anatomiquement, un point directionnel B' de gauche stable anatomiquement, et un point d'induction C' stable anatomiquement, situé en position labiale, du maxillaire inférieur.
